# EUROPEAN PATENT APPLICATION

(11) **EP 4 799 621 A1**
(43) Date of publication of application: **02.09.2026**
(21) Application number: 24881572.2
(22) Date of filing: 22.10.2024
(51) Int. Cl.: A61K 31/438, A61K 31/472, A61K 31/515, A61P 7/06

(54) **LONG-ACTING ORAL PHARMACEUTICAL COMPRISING HYPOXIA-INDUCIBLE FACTOR PROLYL HYDROXYLASE INHIBITOR AND USE THEREOF**

(30) Priority: 24.10.2023 CN 202311391445
(71) Applicant: Kind Pharmaceutical, Hangzhou Zhejiang 311100 (CN)
(72) Inventor: ZHU, Qi, Hangzhou, Zhejiang 311100 (CN); ZHU, Yusha, Hangzhou, Zhejiang 311100 (CN); DU, Ping, Hangzhou, Zhejiang 311100 (CN); LIU, Dong, Hangzhou, Zhejiang 311100 (CN); YU, Pengyang, Hangzhou, Zhejiang 311100 (CN); GU, Danyan, Hangzhou, Zhejiang 311100 (CN); WANG, Wenjuan, Hangzhou, Zhejiang 311100 (CN); ZHU, Peipei, Hangzhou, Zhejiang 311100 (CN); XIONG, Min, Hangzhou, Zhejiang 311100 (CN); DENG, Shaojiang, Hangzhou, Zhejiang 311100 (CN)
(74) Representative: Barker Brettell LLP
(86) International application number: PCT/CN2024/126289
(87) International publication number: WO 2025/087207

(57) **Abstract**

Disclosed are a long-acting oral pharmaceutical comprising a hypoxia-inducible factor prolyl hydroxylase inhibitor (HIF-PHI) and use thereof. Specifically, disclosed is a long-acting oral pharmaceutical formulation comprising a hypoxia-inducible factor prolyl hydroxylase inhibitor (HIF-PHI) or a pharmaceutically acceptable salt thereof, and one or more pharmaceutically acceptable carriers, adjuvants, or excipients. The long-acting oral pharmaceutical formulation is suitable for administration at intervals of once every one week or longer, making it suitable for long-term administration in the treatment of chronic anemia.

## Description

### CROSS-REFERENCE TO RELATED APPLICATION

This application claims priority to Chinese Patent Application No. 202311391445.2 filed on October 24, 2023 and entitled with "long-acting oral pharmaceutical comprising hypoxia-inducible factor prolyl hydroxylase inhibitor and use thereof", which is incorporated herein by reference in its entirety.

### TECHNICAL FIELD

The present application pertains to the medical field, and in particular, to the treatment of diseases such as anemia associated with prolyl hydroxylase.

### BACKGROUND

Anemia is a disease caused by a decrease in the number of erythrocytes or a decrease in erythrocyte hemoglobin content in the body's blood. Because the main function of hemoglobin is to carry oxygen to various organs for use, low levels of hemoglobin will directly lead to insufficient oxygen supply in various body's organs. Since the normal physiological activities of body depend on full utilization of oxygen, various degrees of anemia can cause various clinical symptoms. Common anemia secondary diseases include cardiovascular diseases such as heart failure, atrial fibrillation, angina pectoris and the like, urinary system diseases such as renal failure and proteinuria, and nervous system diseases such as dizziness, headache, tinnitus, vertigo, lack of energy, fatigue and lethargy, irritability, and inattention. Patients with severe anemia may experience fainting, digestive diseases such as loss of appetite and constipation, reproductive system diseases such as decline of sexual desire and irregular menstruation and so on. Not only can anemia seriously affect patient's health and quality of life, but also anemia can even threaten patient's life if it is not improved in time.

There are many causes of anemia, which usually include decreased production of erythropoietin (EPO). Chronic kidney disease (CKD) causes a decrease in erythropoietin synthesis, so most patients with CKD have anemia, i.e. CKD anemia. The pharmacological treatment options for CKD anemia mainly fall into two categories. The first category is based on EPO or Erythropoiesis Stimulating Agents (ESAs) having similar functions, which all require administration by injection. In recent years, another class of oral medications for treating CKD anemia, Hypoxia-Inducible Factor Prolyl Hydroxylase Inhibitors (HIF-PHIs), has been gradually approved by some regulatory agencies. HIF-PHIs are a novel type of small-molecule oral drug for treating renal anemia, which stabilize HIF levels in the body by inhibiting hypoxia-inducible factor prolyl hydroxylase, and regulate multiple downstream target genes, thereby promoting EPO production and achieving therapeutic effects for renal anemia similar to ESAs. Certain HIF-PHIs are disclosed in publications such as WO2004108681, WO2007070359, WO2007150011, WO2008076425, WO2011007856, WO201206472, WO2013043621, WO2014102818, and WO2018205928.

Whether it is the injectable ESAs mentioned above or the oral HIF-PHI drugs, the treatment process for renal anemia essentially involves two stages. The first stage is a dose-fixing period (abbreviated as fixing period), generally not exceeding two months, aimed at raising or adjusting the patient's hemoglobin to the range specified by various regulatory agencies. The second stage is a dose adjustment phase (abbreviated as titration period), which is the routine, long-term maintenance treatment to keep the patient's hemoglobin within the specified regulatory range. This stage is prolonged, requiring patients to take medication long-term.

Research indicates that for chronic diseases requiring long-term management, such as diabetes and chronic kidney disease anemia, treatment outcomes are closely associated with patient adherence to medication. Furthermore, patient adherence is strongly influenced by dosing frequency or dosing interval. For example, For example, studies on drugs that require administration via injection methods such as subcutaneous injection or intramuscular injection, including insulin (Diabetes Spectr 2016, 29(3), 166-170) and GLP-1 agonists (Int J Clin Pract. 2021, 75:e14060), have confirmed that longer dosing intervals correlate with better patient adherence and improved long-term therapeutic outcomes. In the field of chronic kidney disease anemia, research on existing injectable ESAs (Cureus 2020, 12(9): e10358) has also demonstrated that patients show greater adherence to long-acting ESAs with extended dosing intervals, leading to superior long-term efficacy.

For injectable drugs, various techniques exist to extend the interval between their dosing. However, for oral drugs, prolonging their dosing interval presents a significant challenge. There are currently few oral drugs with dosing intervals as long as once weekly or longer, largely due to their relatively short elimination half-lives (t½). For HIF-PHIs used in the treatment of chronic kidney disease anemia, approved or investigational oral drugs are typically administered once daily, including Daprodustat (t½: ~7 hours in patients with chronic kidney disease anemia; see Am J Kidney Dis. 2016, 67(6), 861-871), Vadadustat (t½: 4.7-9.1 hours; see Adv Chronic Kidney Dis. 2019, 26(4), 253-266), Enarodustat (t½: 8.2-8.7 hours; see Drugs 2021, 81, 169-174), and Molidustat (t½: 4-10 hours; see Adv Chronic Kidney Dis. 2019, 26(4), 253-266). Others, such as Roxadustat (t½: 12-15 hours; see Adv Chronic Kidney Dis. 2019, 26(4), 253-266) and Desidustat (t½: 6-15 hours; Drugs 2022, 82(11), 1207-1212), are administered three times weekly.

The dosing regimens of these existing HIF-PHIs universally feature intervals significantly shorter than one week (seven days). For chronic kidney disease anemia requiring lifelong treatment, an oral once-weekly regimen would enhance patient adherence compared to daily or thrice-weekly dosing, thereby facilitating easier disease management. Moreover, patients with chronic kidney disease anemia often have comorbidities such as hypertension, diabetes, hyperphosphatemia, hyperkalemia, hypercholesterolemia, and hyperlipidemia, necessitating the daily intake of multiple other medications. A once-weekly dosing schedule would also simplify avoiding drug-drug interactions between medications for chronic kidney disease anemia and medications for these comorbidities by allowing staggered administration between the medications for chronic kidney disease anemia such as HIF-PHIs and the medications for these comorbidities. In contrast, daily or thrice-weekly regimens would significantly increase the difficulty of timing medications appropriately.

Therefore, there is a critical need for a long-acting oral medication capable of treating chronic anemia, such as chronic kidney disease anemia.

### SUMMARY

The prior art lacks long-acting oral medications for chronic kidney disease anemia. Accoridndg to traditional pharmacological theory, after a patient takes a drug for 5 half-lives (t1/2), the drug is generally considered to be almost completely eliminated from the patient's blood. Therefore, the interval between consecutive doses (dosing interval) for a drug treating a chronic disease usually cannot exceed five elimination half-lives. In practice, to ensure efficacy, dosing intervals exceeding three elimination half-lives are rare. According to the pharmacological theory, if an oral drug is desired to have a dosing interval of no less than once weekly, meaning a dosing cycle of at least one week, then the drug's elimination half-life should not be shorter than 24 hours x 7/5, i.e., the t1/2 must be no less than 33.6 hours. For example, Trelagliptin, an oral DPP-4 inhibitor approved in Japan for treating diabetes with once-weekly dosing, has an elimination half-life (t1/2) of 38.44-54.26 h (J Diabetes Investig 2018, 9, 354-359).

However, the inventors of this application have unexpectedly demonstrated through animal experiments and human clinical trials that for Hypoxia Inducible Factor-Prolyl Hydroxylase Inhibitor (HIF-PHI) drugs, even having a drug's elimination half-life (t1/2) far shorter than 33.6 hours, it is still possible to maintain hemoglobin within the desired range during the titration period of chronic anemia using oral administration once weekly or at even longer intervals. This unexpected discovery allows for the adoption of novel dosing regimens to improve patient adherence to drug therapy for chronic kidney disease anemia, enhance treatment outcomes, and consequently facilitate easier long-term management of the chronic disease.

Therefore, a first aspect of the present application provides a long-acting oral pharmaceutical preparation, comprising a Hypoxia Inducible Factor-Prolyl Hydroxylase Inhibitor (HIF-PHI) or a pharmaceutically acceptable salt thereof, and one or more pharmaceutically acceptable carriers, adjuvants, or excipients, wherein the long-acting oral pharmaceutical preparation is suitable for administration at a dosing interval of once weekly or longer. In particular, the inventors of the present application surprisingly found that during the titration period, a once-weekly dosing frequency reduces side effects compared to a three-times-weekly dosing frequency.

A second aspect of the present application provides a pharmaceutical kit, comprising a packaging container, a mediciation instruction, and a pharmaceutical preparation contained within the packaging container, wherein the pharmaceutical preparation comprises a Hypoxia-Inducible Factor-Prolyl Hydroxylase Inhibitor (HIF-PHI) or a pharmaceutically acceptable salt thereof, and one or more pharmaceutically acceptable carriers, adjuvants, or excipients, and the mediciation instruction contains instructions indicating that the pharmaceutical preparation is suitable for oral administration at a dosing interval of once weekly or longer.

A third aspect of the present application provides use of a Hypoxia Inducible Factor-Prolyl Hydroxylase Inhibitor (HIF-PHI) or a pharmaceutically acceptable salt thereof in the manufacture of a long-acting oral medicament for treating chronic anemia, wherein the long-acting oral medicament is suitable for administration at a dosing interval of once weekly or longer.

A fourth aspect of the present application provides a Hypoxia-Inducible Factor Prolyl Hydroxylase Inhibitor (HIF-PHI) or a pharmaceutically acceptable salt thereof for oral use in treating chronic anemia, wherein the oral treatment is administered at a dosing interval of once weekly or longer.

A fifth aspect of the present application provides use of a Hypoxia-Inducible Factor Prolyl Hydroxylase Inhibitor (HIF-PHI) or a pharmaceutically acceptable salt thereof for treating chronic anemia, wherein the HIF-PHI or pharmaceutically acceptable salt thereof is orally administered at a dosing interval of once weekly or longer.

A sixth aspect of the present application provides a method for treating chronic anemia, comprising: orally administering to a patient in need thereof a therapeutically effective amount of a Hypoxia Inducible Factor-Prolyl Hydroxylase Inhibitor (HIF-PHI) or a pharmaceutically acceptable salt thereof at a dosing interval of once weekly or longer.

### DETAIL DESCRIPTION

### I. Hypoxia Inducible Factor-Prolyl Hydroxylase Inhibitors (HIF-PHIs) or pharmaceutically acceptable salt thereof

In various aspects of the present application and in various embodiments of these aspects, the hypoxia inducible factor-prolyl hydroxylase inhibitor may be various hypoxia inducible factor-prolyl hydroxylase inhibitor compounds known in the art, or may be in the form of a pharmaceutically acceptable salt of the hypoxia inducible factor-prolyl hydroxylase inhibitor compounds. In some embodiments, the HIF-PHI or a pharmaceutically acceptable salt thereof is one having a t1/2 of less than 33.6 hours, less than 24 hours, less than 16 hours, or less than 10 hours.

In some preferred embodiments of the present application, the hypoxia inducible factor-prolyl hydroxylase inhibitor can be an HIF-PHI compound, or a pharmaceutically acceptable salt thereof, that has been approved for clinical use or is undergoing clinical trials. For example, the HIF-PHI can be selected from the group consisting of Roxadustat, Daprodustat, Vadadustat, Molidustat, Enarodustat, Desidustat, HIF117 (SSS17), HEC53856, and DDO-3055.

In some embodiments, the hypoxia inducible factor-prolyl hydroxylase inhibitor (HIF-PHI) is Roxadustat or a pharmaceutically acceptable salt thereof.

In some embodiments, the hypoxia inducible factor-prolyl hydroxylase inhibitor (HIF-PHI) is Daprodustat or a pharmaceutically acceptable salt thereof.

In some embodiments, the hypoxia inducible factor-prolyl hydroxylase inhibitor (HIF-PHI) is Vadadustat or a pharmaceutically acceptable salt thereof.

In some embodiments, the hypoxia inducible factor-prolyl hydroxylase inhibitor (HIF-PHI) is Molidustat or a pharmaceutically acceptable salt thereof.

In some embodiments, the hypoxia inducible factor-prolyl hydroxylase inhibitor (HIF-PHI) is Enarodustat or a pharmaceutically acceptable salt thereof.

In some embodiments, the hypoxia inducible factor-prolyl hydroxylase inhibitor (HIF-PHI) is Desidustat or a pharmaceutically acceptable salt thereof.

In some embodiments, the hypoxia inducible factor-prolyl hydroxylase inhibitor (HIF-PHI) is HIF117 (SSS17) or a pharmaceutically acceptable salt thereof.

In some embodiments, the hypoxia inducible factor-prolyl hydroxylase inhibitor (HIF-PHI) is HEC53856 or a pharmaceutically acceptable salt thereof.

In some embodiments, the hypoxia inducible factor-prolyl hydroxylase inhibitor (HIF-PHI) is DDO-3055 or a pharmaceutically acceptable salt thereof.

In some embodiments, the hypoxia inducible factor-prolyl hydroxylase inhibitor (HIF-PHI) is a compound of Formula I disclosed in WO2018205928, or a pharmaceutically acceptable salt thereof.

In some embodiments, the hypoxia inducible factor-prolyl hydroxylase inhibitor (HIF-PHI) is any mixture of the aforementioned HIF-PHIs.

In the present application, unless otherwise specified, any reference to or designation of a compound or its pharmaceutically acceptable salt by structural formula, name, or code, such as "compound of Formula I" (synonymous with "compound represented by general Formula I"), "Roxadustat", or "(6'-hydroxy-8'-oxo-3'-phenyl-8'H-spiro[cyclopentane-1,5'-indolizine]-7'-carbonyl)glycine", and the like, encompasses various forms of these compounds, including tautomers thereof, optical isomers thereof, geometric isomers thereof, or mixtures of these isomers (e.g., racemic mixtures), and isotopically modified compounds thereof, solvates or hydrates thereof, as well as various solid forms thereof (e.g., various crystalline or amorphous forms thereof).

The term "optical isomer" means that when a compound has one or more chiral centers, each chiral center can exist in the R configuration or the S configuration, and the various isomers constituted thereby are optical isomers. Optical isomers include all diastereomers, enantiomers, mesomers, racemates, or mixtures thereof. For example, optical isomers can be separated by chiral chromatography or by chiral synthesis.

The term "geometric isomer" means that when a double bond exists in a compound, the compound can exist as a cis isomer, trans isomer, E isomer, and Z isomer. Geometric isomers include cis isomers, trans isomers, E isomers, Z isomers, or mixtures thereof.

The term "tautomer" refers to isomers produced by rapid migration of a certain atom within a molecule between two positions. Those skilled in the art can understand that tautomers can interconvert and may reach an equilibrium state in a certain state to achieve coexisting. For example, "compound of Formula I" described herein also encompasses any tautomer of the compound of general Formula I. Specifically, the inventors have found that the compound of Formula I may exist as the following tautomers I-a, I-b, I-c, or I-d:

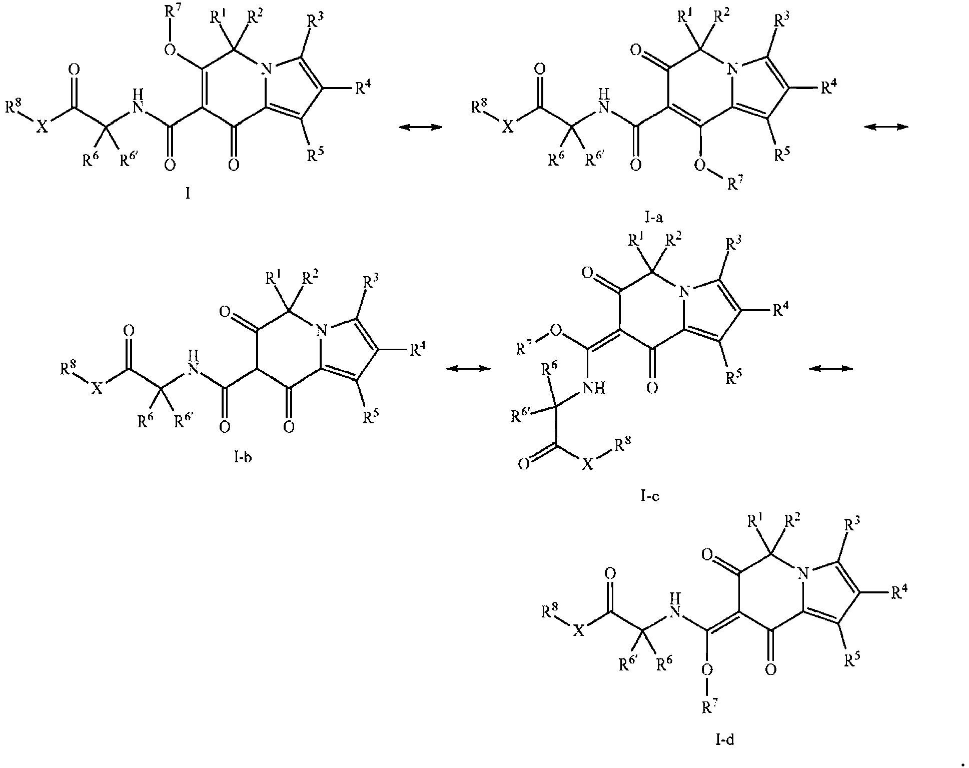

The term "an isotopically modified compound" refers to a compound obtained by replacing any atom in the compound with its isotopic atom. "Isotopically modified compounds" in the present application include all pharmaceutically acceptable isotopically modified compounds of the compound, wherein one or more atoms are replaced by atoms having the same atomic number as those usually found in nature, but with different atomic masses or mass numbers.

Examples of isotopes suitable for inclusion in the compounds of the present application include isotopes of hydrogen, such as ²H (D) and ³H (T); isotopes of carbon, such as ¹¹C, ¹³C, and ¹⁴C; isotopes of chlorine, such as ³⁶Cl; isotopes of fluorine, such as ¹⁸F; isotopes of iodine, such as ¹²³I and ¹²⁵I; isotopes of nitrogen, such as ¹³N and ¹⁵N; isotopes of oxygen, such as ¹⁵O, ¹⁷O, and ¹⁸O; and isotopes of sulfur, such as ³⁵S.

Certain isotopically-labelled compounds of formula (I), for example, those incorporating a radioactive isotope, are useful in drug and/or substrate tissue distribution studies. The radioactive isotopes deuterium, i.e. ²H, tritium, i.e. ³H, and carbon-14, i.e. ¹⁴C, are particularly useful for this purpose in view of their ease of incorporation and ready means of detection.

Substitution with heavier isotopes such as deuterium, i.e. ²H, or tritium, i.e. ³H, may afford certain therapeutic advantages resulting from greater metabolic stability, for example, increased in vivo half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-modified compounds of formula (I) can generally be prepared by conventional techniques known to those skilled in the art or by processes analogous to those described in the accompanying Examples and Preparations using an appropriate isotopically-labeled reagents in place of the non-labeled reagent previously employed.

Certain compounds of the present application may exist in unsolvated form as well as solvated forms, including hydrated forms.

Certain compounds of the present application may exist in different crystalline or amorphous forms, and all such forms are included within the scope of the present application.

In the present application, "pharmaceutically acceptable salt" refers to inorganic or organic acid addition salts, or organic or inorganic base addition salts of the compound that are suitable for in vivo use in mammals (i.e., safe and effective for use). These salts may be prepared in situ during final isolation and purification of the compound, or by separately reacting purified compound in its free form with a suitable organic or inorganic acid or base and isolating the salt thus formed. Typical salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, glucoheptonate, lactobionate, lauryl sulfate, and the like. These salts may include those salts based on cations such as alkali and alkaline earth metals (e.g., sodium, lithium, potassium, calcium, magnesium, etc.), as well as non-toxic ammonium, quaternary ammonium, and amine cations including, but not limited to, ammonium, tetramethylammonium, tetraethylammonium, methylamine, dimethylamine, trimethylamine, triethylamine, ethylamine, etc..

In some preferred embodiments of the present application, the hypoxia inducible factor-prolyl hydroxylase inhibitor (HIF-PHI) is a compound of Formula I or a pharmaceutically acceptable salt thereof disclosed in WO2018205928:

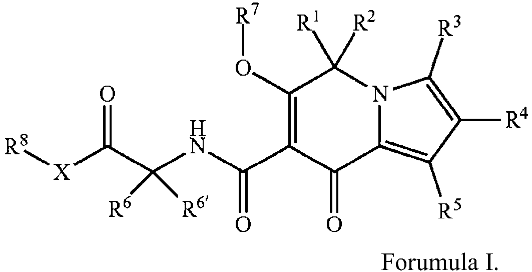

In some embodiments, in the compound represented by general Formula I:
R¹ and R² are independently selected from the group consisting of cyano, alkyl, heterocyclyl, alkenyl, alkynyl, aryl, heteroaryl, and acyl; wherein the alkyl, heterocyclyl, alkenyl, alkynyl, aryl, heteroaryl, and acyl are optionally substituted by one or more substituents independently selected from the group consisting of halogen, cyano, hydroxyl, amino, carboxyl, acyl, alkyl, heterocyclyl, alkenyl, alkynyl, aryl, heteroaryl, =O, =S, SH, R¹⁰O-, R¹⁰S-, R¹⁰ (O=)S-, and R¹⁰ (O=)₂S-, wherein R¹⁰ is alkyl, heterocyclyl, alkenyl, alkynyl, aryl, or heteroaryl; or R₁ and R₂ are taken together to form a ring;
R³ is selected from the group consisting of alkyl, heterocyclyl, alkenyl, alkynyl, aryl, and heteroaryl;
R⁴ and R⁵ are hydrogen;
R⁶ and R⁶' are hydrogen;
R⁷ is hydrogen;
R⁸ is selected from the group consisting of hydrogen and alkyl; and
X is an oxygen atom.

In some embodiments, in the compound represented by general Formula I:
R¹ and R² are independently selected from the group consisting of cyano, C₁-C₁₂ acyclic alkyl, C₂-C₁₂ acyclic alkenyl, C₂-C₁₂ acyclic alkynyl, C₆-C₁₄ aryl, C₃-C₈ cycloalkyl, C₃-C₈ cycloalkenyl, heterocycloalkyl containing 3 to 8 ring atoms, heterocycloalkenyl containing 3 to 8 ring atoms, heteroaryl containing 5 to 14 ring atoms, C₁-C₁₂ acyclic alkyl-C(=O)-, and C₂-C₁₂ acyclic alkenyl-C(=O)-; wherein the C₁-C₁₂ acyclic alkyl, C₂-C₁₂ acyclic alkenyl, C₂-C₁₂ acyclic alkynyl, C₆-C₁₄ aryl, C₃-C₈ cycloalkyl, C₃-C₈ cycloalkenyl, heterocycloalkyl containing 3 to 8 ring atoms, heterocycloalkenyl containing 3 to 8 ring atoms, heteroaryl containing 5 to 14 ring atoms, C₁-C₁₂ acyclic alkyl-C(=O)-, and C₂-C₁₂ acyclic alkenyl-C(=O)-are optionally substituted by 1 to 3 substituents independently selected from the group consisting of hydroxyl, halogen, cyano, amino, carboxyl, C₁-C₆ acyclic alkyl, C₃-C₈ cycloalkyl, C₂-C₆ acyclic alkenyl-, C₂-C₆ acyclic alkynyl-, C₃-C₈ cycloalkyl, C₃-C₈ cycloalkenyl, C₆-C₁₄ aryl, heteroaryl containing 5 to 14 ring atoms, C₁-C₆ acyclic alkyl-O-, C₃-C₈ cycloalkyl-O-, C₂-C₆ acyclic alkenyl-O-, C₂-C₆ acyclic alkynyl-O-, C₃-C₈ cycloalkenyl-O-, C₆-C₁₄ aryl-O-, heteroaryl containing 5 to 14 ring atoms -O-, C₁-C₆ acyclic alkyl-S-, C₃-C₈ cycloalkyl-S-, C₂-C₆ acyclic alkenyl-S-, C₂-C₆ acyclic alkynyl-S-, C₃-C₈ cycloalkenyl-S-, C₆-C₁₄ aryl-S-, heteroaryl containing 5 to 14 ring atoms -S-, heterocycloalkyl containing 3 to 8 ring atoms, heterocycloalkenyl containing 3 to 8 ring atoms, =O, =S, SH, CF₃, -CO₂C₁-C₆ acyclic alkyl, C₁-C₆ acyclic alkyl-S-, C₁-C₆ acyclic alkyl(O=)S-, and C₁-C₆ acyclic alkyl(O=)₂S-; or
R¹ and R² are taken together to form an optionally substituted cycloalkane ring, cycloalkene ring, heterocycloalkane ring, or heterocycloalkene ring containing 3-8 ring atoms;
R³ is selected from the group consisting of C₁-C₁₂ acyclic alkyl, C₂-C₁₂ acyclic alkenyl, C₂-C₁₂ acyclic alkynyl, C₆-C₁₄ aryl, C₃-C₈ cycloalkyl, C₃-C₈ cycloalkenyl, heterocycloalkyl containing 3 to 8 ring atoms, heterocycloalkenyl containing 3 to 8 ring atoms, and heteroaryl containing 5 to 14 ring atoms; wherein the C₁-C₁₂ acyclic alkyl, C₂-C₁₂ acyclic alkenyl, C₂-C₁₂ acyclic alkynyl, C₆-C₁₄ aryl, C₃-C₈ cycloalkyl, C₃-C₈ cycloalkenyl, heterocycloalkyl containing 3 to 8 ring atoms, heterocycloalkenyl containing 3 to 8 ring atoms, and heteroaryl containing 5 to 14 ring atoms are optionally substituted by 1 to 3 substituents independently selected from the group consisting of hydroxyl, halogen, cyano, amino, carboxyl, C₁-C₆ acyclic alkyl, C₃-C₈ cycloalkyl, C₂-C₆ acyclic alkenyl-, C₂-C₆ acyclic alkynyl-, C₃-C₈ cycloalkyl, C₃-C₈ cycloalkenyl, C₆-C₁₄ aryl, heteroaryl containing 5 to 14 ring atoms, C₁-C₆ acyclic alkyl-O-, C₃-C₈ cycloalkyl-O-, C₂-C₆ acyclic alkenyl-O-, C₂-C₆ acyclic alkynyl-O-, C₃-C₈ cycloalkenyl-O-, C₆-C₁₄ aryl-O-, heteroaryl containing 5 to 14 ring atoms -O-, C₁-C₆ acyclic alkyl-S-, C₃-C₈ cycloalkyl-S-, C₂-C₆ acyclic alkenyl-S-, C₂-C₆ acyclic alkynyl-S-, C₃-C₈ cycloalkenyl-S-, C₆-C₁₄ aryl-S-, heteroaryl containing 5 to 14 ring atoms -S-, heterocycloalkyl containing 3 to 8 ring atoms, heterocycloalkenyl containing 3 to 8 ring atoms, =O, =S, SH, CF₃, -CO₂C₁-C₆ acyclic alkyl, C₁-C₆ acyclic alkyl-S-, C₁-C₆ acyclic alkyl(O=)S-, and C₁-C₆ acyclic alkyl(O=)₂S-;
R⁴ and R⁵ are hydrogen;
R⁶ and R⁶' are hydrogen;
R⁷ is hydrogen;
R⁸ is selected from the group consisting of hydrogen, C₁-C₁₂ acyclic alkyl, and C₃-C₈ cycloalkyl; wherein the C₁-C₁₂ acyclic alkyl, and C₃-C₈ cycloalkyl are optionally substituted by 1 to 3 substituents independently selected from the group consisting of hydroxyl, halogen, cyano, amino, carboxyl, C₁-C₆ acyclic alkyl, C₃-C₈ cycloalkyl, C₂-C₆ acyclic alkenyl-, C₂-C₆ acyclic alkynyl-, C₃-C₈ cycloalkyl, C₃-C₈ cycloalkenyl, C₆-C₁₄ aryl, heteroaryl containing 5 to 14 ring atoms, C₁-C₆ acyclic alkyl-O-, C₃-C₈ cycloalkyl-O-, C₂-C₆ acyclic alkenyl-O-, C₂-C₆ acyclic alkynyl-O-, C₃-C₈ cycloalkenyl-O-, C₆-C₁₄ aryl-O-, and heteroaryl containing 5 to 14 ring atoms -O-; and
X is an oxygen atom.

In some preferred embodiments of the present application, in the compound represented by Formula I, R¹ and R² are independently selected from the group consisting of cyano and unsubstituted C₁-C₆ acyclic alkyl; or R¹ and R² are taken together to form an optionally substituted cycloalkane ring or heterocycloalkane ring containing 3-8 ring atoms.

In some preferred embodiments of the present application, in the compound represented by Formula I, R¹ and R² are independently selected from the group consisting of methyl and ethyl; or R¹ and R² are taken together to form a cyclopropane ring, a cyclobutane ring, a cyclopentane ring, a cyclohexane ring, a methylcyclohexane ring, a cycloheptane ring, a tetrahydrofuran ring, or a tetrahydropyran ring.

In some preferred embodiments of the present application, in the compound represented by Formula I, R¹ and R² are independently selected from the group consisting of cyano and unsubstituted C₁-C₆ acyclic alkyl.

In some preferred embodiments of the present application, in the compound represented by Formula I, R¹ and R² are independently selected from the group consisting of methyl and ethyl.

In some preferred embodiments of the present application, in the compound represented by Formula I, R¹ and R² are taken together to form an optionally substituted heterocycloalkane ring containing 3-8 ring atoms.

In some preferred embodiments of the present application, in the compound represented by Formula I, R¹ and R² are taken together to form a tetrahydrofuran ring or a tetrahydropyran ring.

In some preferred embodiments of the present application, in the compound represented by Formula I, R¹ and R² are taken together to form an optionally substituted cycloalkane ring containing 3-8 ring atoms.

In some preferred embodiments of the present application, in the compound represented by Formula I, R¹ and R² are taken together to form a cyclopropane ring, a cyclobutane ring, a cyclopentane ring, a cyclohexane ring, a methylcyclohexane ring, or a cycloheptane ring.

In some preferred embodiments of the present application, in the compound represented by Formula I, R³ is selected from the group consisting of C₁-C₆ acyclic alkyl, C₆-C₁₄ aryl, C₃-C₈ cycloalkyl, and heteroaryl containing 5 to 14 ring atoms; wherein the C₁-C₆ acyclic alkyl, C₆-C₁₄ aryl, and C₃-C₈ cycloalkyl are optionally substituted by 1 to 3 substituents independently selected from the group consisting of hydroxyl, halogen, cyano, amino, carboxyl, C₆-C₁₄ aryl, and C₁-C₆ acyclic alkyl.

In some preferred embodiments of the present application, in the compound represented by Formula I, R³ is selected from the group consisting of methyl, ethyl, propyl, butyl, phenyl, benzyl, tolyl, methoxyphenyl, chlorophenyl, fluorophenyl, bromophenyl, cyclopropyl, cyclobutyl, cyclopentyl, cyclohexyl, and pyridyl.

In some preferred embodiments of the present application, in the compound represented by Formula I, R³ is C₁-C₆ acyclic alkyl; wherein the C₁-C₆ acyclic alkyl is optionally substituted by 1 to 3 substituents independently selected from the group consisting of hydroxyl, halogen, cyano, amino, carboxyl, C₆-C₁₄ aryl, and C₁-C₆ acyclic alkyl.

In some preferred embodiments of the present application, in the compound represented by Formula I, R³ is selected from the group consisting of methyl, ethyl, propyl, and butyl.

In some preferred embodiments of the present application, in the compound represented by Formula I, R³ is C₆-C₁₄ aryl; wherein the C₆-C₁₄ aryl is optionally substituted by 1 to 3 substituents independently selected from the group consisting of hydroxyl, halogen, cyano, amino, carboxyl, C₆-C₁₄ aryl, and C₁-C₆ acyclic alkyl.

In some preferred embodiments of the present application, in the compound represented by Formula I, R³ is selected from the group consisting of phenyl, benzyl, tolyl, methoxyphenyl, chlorophenyl, fluorophenyl, and bromophenyl, preferably phenyl.

In some preferred embodiments of the present application, in the compound represented by Formula I, R³ is C₃-C₈ cycloalkyl; wherein the C₃-C₈ cycloalkyl is optionally substituted by 1 to 3 substituents independently selected from the group consisting of hydroxyl, halogen, cyano, amino, carboxyl, C₆-C₁₄ aryl, and C₁-C₆ acyclic alkyl.

In some preferred embodiments of the present application, in the compound represented by Formula I, R³ is selected from the group consisting of cyclopropyl, cyclobutyl, cyclopentyl, and cyclohexyl.

In some preferred embodiments of the present application, in the compound represented by Formula I, R³ is heteroaryl containing 5 to 14 ring atoms.

In some preferred embodiments of the present application, in the compound represented by Formula I, R³ is pyridyl.

In some preferred embodiments of the present application, in the compound represented by Formula I, R⁸ is selected from the group consisting of hydrogen and C₁-C₆ acyclic alkyl; wherein the C₁-C₆ acyclic alkyl is optionally substituted by 1 to 3 substituents independently selected from the group consisting of hydroxyl, halogen, cyano, amino, carboxyl, C₁-C₆ acyclic alkyl, and C₁-C₆ acyclic alkyl-O-.

In some preferred embodiments of the present application, in the compound represented by Formula I, R⁸ is selected from the group consisting of hydrogen, methyl, ethyl, propyl, butyl, and pentyl.

In some preferred embodiments of the present application, in the compound represented by Formula I, R⁸ is hydrogen.

In some preferred embodiments of the present application, in the compound represented by Formula I, R⁸ is C₁-C₆ acyclic alkyl; wherein the C₁-C₆ acyclic alkyl is optionally substituted by 1 to 3 substituents independently selected from the group consisting of hydroxyl, halogen, cyano, amino, carboxyl, C₁-C₆ acyclic alkyl, and C₁-C₆ acyclic alkyl-O-.

In some preferred embodiments of the present application, in the compound represented by Formula I, R⁸ is selected from the group consisting of methyl, ethyl, propyl, butyl, and pentyl.

In some preferred embodiments of the present application, in the compound represented by Formula I, R¹ and R² are taken together to form an optionally substituted cycloalkane ring containing 3-8 ring atoms; R³ is C₆-C₁₄ aryl; wherein the C₆-C₁₄ aryl is optionally substituted by 1 to 3 substituents independently selected from the group consisting of hydroxyl, halogen, cyano, amino, carboxyl, C₆-C₁₄ aryl, and C₁-C₆ acyclic alkyl; and R⁸ is selected from the group consisting of hydrogen and C₁-C₆ acyclic alkyl; wherein the C₁-C₆ acyclic alkyl is optionally substituted by 1 to 3 substituents independently selected from the group consisting of hydroxyl, halogen, cyano, amino, carboxyl, C₁-C₆ acyclic alkyl, and C₁-C₆ acyclic alkyl-O-.

In some preferred embodiments of the present application, in the compound represented by Formula I, R¹ and R² are each independently selected from C₁-C₆ acyclic alkyl, wherein the C₁-C₆ acyclic alkyl is optionally substituted by 1 to 3 substituents of C₁-C₆ acyclic alkyl; or R¹ and R² are taken together to form a cyclopropane ring, a cyclobutane ring, a cyclopentane ring, a cyclohexane ring, a methylcyclohexane ring, a cycloheptane ring, or a tetrahydropyran ring; R³ is C₆-C₁₄ aryl; wherein the C₆-C₁₄ aryl is optionally substituted by 1 to 3 substituents independently selected from the group consisting of hydroxyl, halogen, cyano, amino, carboxyl, C₆-C₁₄ aryl, and C₁-C₆ acyclic alkyl; and R⁸ is selected from the group consisting of hydrogen and C₁-C₆ acyclic alkyl; wherein the C₁-C₆ acyclic alkyl is optionally substituted by 1 to 3 substituents independently selected from the group consisting of hydroxyl, halogen, cyano, amino, carboxyl, C₁-C₆ acyclic alkyl, and C₁-C₆ acyclic alkyl-O-.

In some preferred embodiments of the present application, in the compound represented by Formula I, R¹ and R² are taken together to form a cyclopropane ring, a cyclobutane ring, a cyclopentane ring, a cyclohexane ring, a methylcyclohexane ring, or a cycloheptane ring; R³ is C₆-C₁₄ aryl; wherein the C₆-C₁₄ aryl is optionally substituted by 1 to 3 substituents independently selected from the group consisting of hydroxyl, halogen, cyano, amino, carboxyl, C₆-C₁₄ aryl, and C₁-C₆ acyclic alkyl; and R⁸ is hydrogen.

In some particularly preferred embodiments of the present application, the compound represented by Formula I is (6'-hydroxy-8'-oxo-3'-phenyl-8'H-spiro[cyclopentane-1,5'-indolizine]-7'-carbonyl)glycine. That is, the compound is those where R¹ and R² together with a carbon atom to which they are attached form a cyclopentane ring (meaning R¹ and R² are - CH₂CH₂CH₂CH₂-), R³ is phenyl, R⁴ and R⁵ are hydrogen, R⁶ and R⁶' are hydrogen, R⁷ is hydrogen, R⁸ is hydrogen, and X is oxygen.

In the various preferred embodiments above, the preferred options for each substituent can be combined with each other, and all such combinations are within the scope of the present application.

To avoid ambiguity, the definitions of the terms used herein are given below. Unless otherwise stated, the meanings of the terms used herein are as follows.

The term "hydroxy" refers to -OH.

The term "halogen" or "halo" refers to -F, -Cl, -Br, or -I.

The term "amino" refers to -NH₂.

The term "cyano" refers to -CN.

The term "carboxy" refers to -C(=O)OH.

The term "substituted" means that one or more (preferably 1 to 5, more preferably 1 to 3) hydrogen atoms in a group are independently replaced by a corresponding number of substituents.

The term "independently" means that when the number of substituents is more than one, these substituents may be the same or different.

The term "optional" or "optionally" means that the event described therein may or may not occur. For example, an "optionally substituted" group means that the group may be unsubstituted or substituted.

The term "heteroatom" as used herein refers to oxygen (O), nitrogen (N), or S(O)ₘ in which m may be 0, 1 or 2, i.e. a sulfur atom S, or a sulfoxide group SO, or a sulfonyl group S(O)₂).

The term "alkyl" refers to a group formed by removing a hydrogen atom at any carbon atom from a saturated hydrocarbon consisting solely of two elements, C and H. The "alkyl group" described herein includes an acyclic alkyl group such as a linear alkyl group or a branched alkyl group; and a cycloalkyl group such as a monocyclic alkyl group, a spirocycloalkyl group, a fused cycloalkyl group, or a bridged cycloalkyl group. Preferably, in the present application, the "alkyl" refers to an acyclic alkyl group. The alkyl group may be unsubstituted or substituted.

The "alkyl" as used herein includes an optionally substituted acyclic alkyl group which preferably has from 1 to 20 carbon atoms, more preferably from 1 to 12 carbon atoms, and most preferably from 1 to 6 carbon atoms; for example, methyl, ethyl, n-propyl, isopropyl, n-butyl, sec-butyl, isobutyl, tert-butyl, n-pentyl, n-hexyl, chloromethyl, fluoroethyl, trifluoromethyl or 1,1,1-trifluoroethyl and the like.

The "alkyl" as used herein also includes an optionally substituted cycloalkyl (e.g., C3-C20 cycloalkyl or C3-C12 cycloalkyl or C3-C8 cycloalkyl), for example, cyclopropyl, cyclobutyl, cyclopentyl, cyclohexyl, cycloheptyl, cyclooctyl, decalinyl, norbornyl, adamantyl, fluorocyclopropyl, 2-iodocyclobutyl, 2,3-dimethyl cyclopentyl, 2,2-dimethoxycyclohexyl and 3-phenylcyclopentyl and the like.

The "C1-C6 acyclic alkyl", also known as "lower acyclic alkyl", is a subset of alkyl which refers to a linear or branched alkyl group having from 1 to 6 carbon atoms, including, for example, methyl, ethyl, n-propyl, isopropyl, n-butyl, sec-butyl, isobutyl, tert-butyl, n-pentyl, n-hexyl, and the like.

The "alkyl" as used herein is optionally substituted by one or more substituents, wherein the substituents are independently selected from the group consisting of halo, cyano, nitro (-NO₂), hydroxy, amino, carboxy, acyl, alkyl, heterocyclyl, alkenyl, alkynyl, aryl, heteroaryl, =O, =S, -SH, R¹⁶O-, R¹⁶S-, R¹⁶(O=)S-, and R¹⁶(O=)₂S-, wherein R¹⁶ is alkyl, heterocyclyl, alkenyl, alkynyl, aryl or heteroaryl.

Preferably, the "alkyl" as used herein is optionally substituted with from 1 to 3 substituents, wherein the substituents are independently selected from the group consisting of hydroxy, halo, nitro, cyano, amino, carboxy, C1-C6 acyclic alkyl, C3-C8 cycloalkyl, C2-C6 acyclic alkenyl-, C2-C6 acyclic alkynyl-, C3-C8 cycloalkyl, C3-C8 cycloalkenyl, C6-C14 aryl, heteroaryl having 5 to 14 ring members, C1-C6 acyclic alkyl-O-, C3-C8 cycloalkyl-O-, C2-C6 acyclic alkenyl-O-, C2-C6 acyclic alkynyl-O-, C3-C8 cycloalkenyl-O-, C6-C14 aryl-O-, heteroaryl-O- having 5 to 14 ring members, C1-C6 acyclic alkyl-S-, C3-C8 cycloalkyl-S-, C2-C6 acyclic alkenyl-S-, C2-C6 acyclic alkynyl-S-, C3-C8 cycloalkenyl-S-, C6-C14 aryl-S-, heteroaryl-S- having 5 to 14 ring members, heterocycloalkyl having 3 to 8 ring members, heterocycloalkenyl having 3 to 8 ring members, =O, =S, -SH, -CF₃, -CO₂C₁-C₆ acyclic alkyl group, C1-C6 acyclic alkyl-S-, C1-C6 acyclic alkyl (O=)S- and C1-C6 acyclic alkyl (O=)₂S-.

The term "alkenyl" refers to a group formed by removing a hydrogen atom at any carbon atom from a hydrocarbon that consists of only two elements, C and H, and which contains one or more carbon-carbon double bonds without carbon-carbon triple bonds or aromatic bonds. The "alkenyl" as used herein includes an acyclic alkenyl group such as a linear or branched alkenyl group; and also includes a cyclic alkenyl group such as a monocycloalkenyl group, a spirocycloalkenyl group, a fused cycloalkenyl group or a bridged cycloalkenyl group. The alkenyl group may be unsubstituted or substituted.

The "alkenyl" as used herein includes an optionally substituted acyclic alkenyl group, preferably having from 2 to 20 carbon atoms, more preferably from 2 to 12 carbon atoms, most preferably from 2 to 6 carbon atoms; for example, vinyl, 1-propenyl, 2-propenyl, 1-butenyl, 2-butenyl, isobutenyl, 1-pentenyl, 2-pentenyl, isopentenyl, 1-hexenyl, 2-hexenyl, 3-hexenyl, isohexenyl, 1-heptenyl, 2-heptenyl, 3-heptenyl, 1-octenyl, 2-octenyl, 3-octenyl, 4-octenyl, 1-nonenyl, 1-decenyl, 1-undecenyl, 1-dodecenyl, and the like.

The "alkenyl" as used herein also includes an optionally substituted cycloalkenyl (e.g., C3-C20 cycloalkenyl or C3-C12 cycloalkenyl or C3-C8 cycloalkenyl), for example, cyclobutenyl, cyclopentenyl, cyclohexenyl, cycloheptenyl, cyclobutadienyl, cyclopentadienyl, cycloheptatrienyl, and the like.

The "alkenyl" as used herein is optionally substituted by one or more (e.g., 1-3) substituents, wherein the choice and preference of the substituents are the same as those for the "alkyl".

The term "alkynyl" refers to a group formed by removing a hydrogen atom at any carbon atom from a hydrocarbon that consists of only two elements C and H and which contains one or more carbon-carbon triple bonds without aromatic bonds. The "alkynyl" as used herein includes an acyclic alkynyl group such as a linear or branched alkynyl group, and includes a cycloalkynyl group such as monocycloalkynyl, spirocycloalkynyl, fused cycloalkynyl, or bridged alkynyl. The alkynyl group can optionally contain one or more carbon-carbon double bonds. The alkynyl group may be unsubstituted or substituted.

As used herein, "alkynyl" includes an optionally substituted acyclic alkynyl group, preferably having from 2 to 20 carbon atoms, more preferably from 2 to 12 carbon atoms, most preferably from 2 to 6 carbon atoms; for example, acetynyl, propynyl, 1-butynyl, 2-butynyl, isobutynyl, 1-pentynyl, 2-pentynyl, isopenynyl, 3-methyl-3-butynyl , 1-hexynyl, 2-hexynyl, 3-hexynyl, 3-heptynyl, 1-octynyl, 1-nonynyl, 1-decynyl, 1-undecynyl , 1-dodecynyl and the like.

The "alkynyl" as used herein also includes optionally substituted cycloalkynyl (e.g., C8-C18 cycloalkynyl), for example, cyclooctynyl, and the like.

The "alkynyl" as used herein is optionally substituted by one or more (e.g., 1-3) substituents, wherein the choice and preference of the substituents are the same as those for the "alkyl".

The term "heterocyclyl" refers to a group derived from a monocyclic or polycyclic compound that is saturated or contains a carbon-carbon double bond or a carbon-carbon triple bond; which group contains 3 to 20 ring members (preferably 3 to 12 ring members, more preferably 3 to 8 ring members), wherein one or more ring members are selected from heteroatoms, and the remaining ring members are carbon; and any one of the rings has no aromaticity. The "heterocyclyl" as used herein also includes spiroheterocyclyl, fused heterocyclyl and bridged heterocyclyl. The heterocyclyl may be unsubstituted or substituted. The heterocyclyl group may be a heterocycloalkyl group, a heterocycloalkenyl group or a heterocycloalkynyl group. Examples of suitable monocyclic heterocyclcyl include, but are not limited to, piperidinyl, pyrrolidinyl, piperazinyl, azetidinyl, azacyclopropyl, morpholinyl, thietanyl, oxacyclopentyl (tetrahydrofuranyl), oxacyclohexyl (tetrahydropyranyl) and the like.

It will be understood that the "heterocyclyl" as used herein is optionally substituted by one or more (e.g., one to three) substituents, wherein the choice and preference of the substituents are the same as those for the "alkyl".

The term "aryl" refers to a group having a conjugated pi-electron system derived from 6 to 14 membered pure carbon monocyclic or fused polycyclic compound. The aryl ring may be fused to a heteroaromatic ring, a heterocyclic ring, cycloalkane, spirocycloalkane, fused cycloalkane, bridged cycloalkane, cycloalkenylene, spirocycloalkene, fused cycloalkene, bridged cycloalkene, cycloalkyne, spirocycloalkyne, fused cycloalkyne or bridged cycloalkyne. The aryl group may be unsubstituted or substituted. Examples thereof include, but are not limited to, phenyl, naphthyl, 2-methylphenyl, 3-methylphenyl, 4-methylphenyl, methoxyphenyl (such as 2-methoxyphenyl, 3 -methoxyphenyl, 4-methoxyphenyl), chlorophenyl (such as 2-chlorophenyl, 3-chlorophenyl, 4-chlorophenyl), fluorophenyl (such as 2-fluorophenyl, 3-fluorophenyl, 4-fluorophenyl), bromophenyl (such as 2-bromophenyl, 3-bromophenyl, 4-bromophenyl), 2-chloro-3-methylphenyl, 2-chloro-4-methylphenyl, 2-chloro-5-methylphenyl, 3-chloro-2-methylphenyl, 3-chloro-4-methylphenyl, 4-chloro-2-methyl phenyl, 4-chloro-3-methylphenyl, 5-chloro-2-methylphenyl, 2,3-dichlorophenyl, 2,5-dichlorophenyl, 3,4-dichlorophenyl, 2,3-dimethylphenyl, 3,4-dimethylphenyl, and the like.

The "aryl" as used herein are optionally substituted with from 1 to 4 or from 1 to 3 substituents, wherein the choice and preference of the substituents are the same as those for the "alkyl".

The term "heteroaryl" refers to a group derived from an aromatic system containing from 5 to 18 ring members, preferably from 5 to 14 ring members, one or four ring members of which are heteroatoms selected from the group consisting of oxygen, nitrogen and sulfur. The heteroaryl ring may be fused to an aryl ring, a heterocyclic ring, cycloalkane, spirocycloalkane, fused cycloalkane, bridged cycloalkane, cycloalkenylene, spirocycloalkene, fused cycloalkene, bridged cycloalkene, cycloalkyne, spirocycloalkyne, fused cycloalkyne or bridged cycloalkyne. The "heteroaryl" may be unsubstituted or substituted. Examples of heteroaryl groups include, but are not limited to, thienyl, furanyl, pyrrolyl, pyridyl, pyrimidinyl, imidazolyl, pyrazinyl, oxazolyl, thiazolyl, benzothienyl, benzofuranyl, benzooxazolyl, benzimidazolyl, indenyl, quinolyl, isoquinolyl and quinazolinyl, and the like.

The "heteroaryl" as used herein are optionally substituted with from 1 to 4 or from 1 to 3 substituents, wherein the choice and preference of the substituents are the same as those for the "alkyl".

The term "acyl" as used herein refers to RC(=O)-, wherein R is C1-C18 (preferably C1-C12, more preferably C1-C6) alkyl. Examples of "acyl" include, but are not limited to, formyl, acetyl, benzoyl, nicotinyl, propionyl, isobutyryl, oxalyl, and the like.

The acyl group RC(=O)- as used herein is optionally substituted by one or more (e.g., 1-3) substituents, wherein the choice and preference of the substituents are the same as those for the "alkyl".

The term "form a ring" as used herein means forming a cyclic structure such as a cycloalkane ring, a cycloalkene ring, a cycloalkyne ring, an aromatic ring, a heterocycloalkane ring, a heterocycloalkene ring, a heterocycloalkyne ring, a heteroaryl ring or the like wherein the cyclic structure may be a monocyclic, bicyclic or polycyclic structure including its fused ring, bridged ring, and spiro ring structure. Particularly, the ring formed by the substituents R¹ and R² herein is preferably a 3- to 12-membered ring, particularly preferably a 3- to 12-membered cycloalkane ring, cycloalkene ring, heterocycloalkane ring, and heterocycloalkene ring, and most preferably a 3- to 8-membered cycloalkane ring, cycloalkene ring, heterocycloalkane ring, and heterocycloalkene ring, such as a cyclopropane ring, a cyclobutane ring, a cyclopentane ring, a cyclohexane ring, a cycloheptane ring, a tetrahydrofuran ring, a tetrahydropyran ring and the like. The ring structure is optionally substituted with one or more (e.g., 1-3) substituents, wherein the choice and preference of the substituents are the same as those for the "alkyl".

Herein, a numerical range relating to the number of substituents, the number of carbon atoms, and the number of ring members represents an enumeration of all integers in the range, and the range is only a simplified representation thereof. For example:
"1-4 substituents" means 1, 2, 3 or 4 substituents;
"1-3 substituents" means a 1, 2 or 3 substituent;
"3 to 12-membered ring" means a 3, 4, 5, 6, 7, 8, 9, 10, 11 or 12-membered ring;
"3 to 8 membered ring" means a 3, 4, 5, 6, 7, or 8 membered ring;
"1-12 carbon atoms" or "C1-C12" means 1 (C1), 2 (C2), 3 (C3), 4 (C4), 5 (C5), 6 (C6) , 7 (C7), 8 (C8), 9 (C9), 10 (C10), 11 (C11) or 12 (C12) carbon atoms;
"1-6 carbon atoms" or "C1-C6" means 1 (C1), 2 (C2), 3 (C3), 4 (C4), 5 (C5) or 6 (C6) carbon atoms;
"2-6 carbon atoms" or "C2-C6" means 2 (C2), 3 (C3), 4 (C4), 5 (C5) or 6 (C6)carbon atoms;
"C3-C8" means 3 (C3), 4 (C4), 5 (C5), 6 (C6), 7 (C7) or 8 (C8) carbon atoms;
"3 to 8 ring members" means 3, 4, 5, 6, 7, or 8 ring members.

Thus, a numerical range associated with the number of substituents, the number of carbon atoms, and the number of ring members also encompasses any one of its subranges, and each subrange is also considered to be disclosed herein.

In some particularly preferred embodiments of the present application, the hypoxia inducible factor-prolyl hydroxylase inhibitor (HIF-PHI) is the compound (6'-hydroxy-8'-oxo-3'-phenyl-8'H-spiro[cyclopentane-1,5'-indolizine]-7'-carbonyl)glycine disclosed in WO2018205928, or a pharmaceutically acceptable salt thereof. The compound (6'-hydroxy-8'-oxo-3'-phenyl-8'H-spiro[cyclopentane-1,5'-indolizine]-7'-carbonyl)glycine is the compound of Formula I wherein R₁ and R₂ together with a caron atom to which they are attached form a cyclopentane ring (i.e., -CH₂CH₂CH₂CH₂-), R₃ is phenyl, R₄ and R₅ are hydrogen, R₆ and R₆' are hydrogen, R₇ is hydrogen, R₈ is hydrogen, and X is an oxygen atom.

### 2. Pharmaceutical Preparation

A first aspect of the present application provides a long-acting oral pharmaceutical preparation, comprising a hypoxia inducible factor-prolyl hydroxylase inhibitor (HIF-PHI) or a pharmaceutically acceptable salt thereof, and one or more pharmaceutically acceptable carriers, adjuvants, or excipients, wherein the long-acting oral pharmaceutical preparation is suitable for administration at a dosing interval of once weekly or longer. In the context of the present application, the term "a long-acting oral pharmaceutical preparation" refers to an oral pharmaceutical preparation that, under the premise of achieving the desired therapeutic effect, allows for administration at a dosing interval of once weekly or longer.

For therapeutic applications, HIF-PHIs are usually administered to a patient in the form of a pharmaceutical composition comprising at least one above-mentioned compound as an active ingredient, optionally together with a pharmaceutically acceptable adjuvant and/or excipient, and a pharmaceutically acceptable solid or liquid carrier.

The pharmaceutical composition of the present application can be formulated into various pharmaceutical dosage forms suitable for oral administration as needed. Thus, if a solid carrier is used, the preparation can be in the form of a tablet, a hard gelatin capsule where the active ingredient is present in powder or granular form, or a troche or lozenge. Solid carriers can include conventional excipients such as binders, fillers, tableting lubricants, disintegrants, wetting agents, and the like. The tablets can be film-coated by conventional techniques if desired. If a liquid carrier is used, the preparation can be in the form of a syrup, emulsion, soft gelatin capsule, aqueous or non-aqueous liquid suspension, or can be a dry product for reconstitution with water or other suitable vehicle before use. Liquid preparations may contain conventional additives such as suspending agents, emulsifying agents, wetting agents, non-aqueous vehicles (including edible oils), preservatives, and flavoring and/or coloring agents.

These pharmaceutical compositions (or pharmaceutical preparations) may also contain various excipients, for example, preservatives, wetting agents, emulsifying agents, and dispersing agents. Inhibition of action of microorganisms can be ensured by various antibacterial and antifungal agents, for example, parabens, chlorobutanol, phenol, sorbic acid, and the like. It may also be desirable to include isotonic agents, for example, sugars, sodium chloride, and the like. Prolonged absorption of an injectable pharmaceutical form can be brought about by use of agents delaying absorption, for example, aluminum monostearate and gelatin.

Solid dosage forms for oral administration include capsules, tablets, pills, powders, and granules. In such solid dosage forms, the active compound is mixed with at least one inert excipient (or carrier), such as sodium citrate or dicalcium phosphate, and/or with (a) fillers or extenders such as starches, lactose, sucrose, glucose, mannitol, and silicic acid; (b) binders such as carboxymethylcellulose, alginates, gelatin, polyvinylpyrrolidone, sucrose, and acacia; (c) humectants such as glycerol; (d) disintegrating agents such as agar-agar, calcium carbonate, potato or tapioca starch, alginic acid, certain complex silicates, and sodium carbonate; (e) solution retarders such as paraffin; (f) absorption accelerators such as quaternary ammonium compounds; (g) wetting agents such as cetyl alcohol and glycerol monostearate; (h) adsorbents such as kaolin and bentonite; and (i) lubricants such as talc, calcium stearate, magnesium stearate, solid polyethylene glycols, sodium lauryl sulfate, or mixtures thereof.

In soft and hard-filled gelatin capsules, similar types of solid pharmaceutical compositions (or pharmaceutical preparations) may also be employed as fillers using excipients such as lactose and high molecular weight polyethylene glycols, and the like.

Solid dosage forms such as tablets, dragees, capsules, pills, and granules can be prepared with coatings and shells such as enteric coatings and others well known in the art. They may contain opacifying agents, and may also be of such composition that they release the active compound or compounds in a delayed manner in a certain part of the intestinal tract. The active components can also be in micro-encapsulated form, if appropriate, with one or more of the above-mentioned excipients.

Liquid dosage forms for oral administration include pharmaceutically acceptable emulsions, solutions, dispersions, syrups, and elixirs. In addition to the active compounds, liquid dosage forms may contain inert diluents commonly used in the art, such as water or other solvents, solubilizing agents and emulsifiers such as ethanol, isopropanol, ethyl carbonate, ethyl acetate, benzyl alcohol, benzyl benzoate, propylene glycol, 1,3-butylene glycol, and dimethylformamide, oils such as cottonseed oil, groundnut oil, corn oil, olive oil, castor oil, and sesame oil, glycerol, tetrahydrofurfuryl alcohol, polyethylene glycols, and fatty acid esters of sorbitan, or mixtures of these substances, and the like.

Besides such inert diluents, the pharmaceutical compositions (or pharmaceutical preparations) can also include adjuvants such as wetting agents, emulsifying and suspending agents, sweetening, flavoring, and perfuming agents.

In addition to the active compounds, suspensions may contain suspending agents such as ethoxylated isostearyl alcohols, polyoxyethylene sorbitol, sorbitan esters, microcrystalline cellulose, aluminum metahydroxide, bentonite, agar-agar, and tragacanth, or mixtures of these substances, and the like.

The amount of the HIF-PHI compound in the pharmaceutical composition (or pharmaceutical preparation) can be appropriately determined by a person skilled in the art as needed.

In some embodiments, the HIF-PHI or a pharmaceutically acceptable salt thereof is present in each unit pharmaceutical preparation in an amount of: about 1-1000 mg, about 1-10 mg, about 10 mg, about 5 mg, about 4.5 mg, about 1-3 mg, about 2-4 mg, about 3-5 mg, about 4-6 mg, about 5-7 mg, about 6-8 mg, about 7-9 mg, about 8-10 mg, about 9-11 mg, about 10-12 mg, about 4.5-5 mg, 20 mg, 30 mg, 30-100 mg, about 40 mg, about 50 mg, about 60 mg, about 70 mg, about 80 mg, about 90 mg, about 10-30 mg, about 30-50 mg, about 50-70 mg, about 10-90 mg, about 10-800 mg, about 10-700 mg, about 10-600 mg, about 10-500 mg, about 10-400 mg, about 10-300 mg, or about 10-200 mg, or in an amount within any range defined by any of these values.

In some embodiments, the HIF-PHI or a pharmaceutically acceptable salt thereof is present in each unit pharmaceutical preparation in an amount of: about 0.01-0.2 mg, about 0.2-0.4 mg, about 0.4-0.6 mg, about 0.6-0.8 mg, about 0.8-1 mg, about 1-1.2 mg, about 1.2-1.4 mg, about 1.4-1.6 mg, about 1.6-1.8 mg, about 1.8-2 mg, about 2-2.2 mg, about 2.2-2.4 mg, about 2.4-2.6 mg, about 2.6-2.8 mg, about 2.8-3 mg, about 3-3.2 mg, about 3.2-3.4 mg, about 3.4-3.6 mg, about 3.6-3.8 mg, about 3.8-4 mg, about 3.9-4.1 mg, about 4-4.2 mg, about 0.2-0.4 mg, about 0.2-0.6 mg, about 0.2-0.8 mg, about 0.2-1 mg, about 0.2-1.2 mg, about 0.2-1.4 mg, about 0.2-1.6 mg, about 0.2-1.8 mg, about 0.2-2.0 mg, 0.2-2.5 mg, about 0.2-3.0 mg, about 0.2-3.5 mg, about 0.2-4.0 mg, about 5-10 mg, about 10-15 mg, about 15-20 mg, about 20-25 mg, about 25-30 mg, about 30-40 mg, about 40-50 mg, about 50-60 mg, about 60-70 mg, about 70-80 mg, about 80-90 mg, about 90-100 mg, about 100-120 mg, about 120-140 mg, about 140-150 mg, about 150-160 mg, about 160-180 mg, about 180-200 mg, about 200-220 mg, about 220-240 mg, about 10-500 mg, about 50-400 mg, about 50-300 mg, about 100-250 mg, about 1-10 mg, about 10-200 mg, about 10-150 mg, about 10-100 mg, about 10-180 mg, about 10-160 mg, about 10-140 mg, about 10-120 mg, about 10-100 mg, about 10-20 mg, about 20-30 mg, about 30-40 mg, about 40-50 mg, about 50-60 mg, about 60-70 mg, about 70-80 mg, about 80-90 mg, about 90-100 mg, about 100-120 mg, about 120-140 mg, about 140-160 mg, about 160-180 mg, about 180-200 mg, about 200-220 mg, about 220-240 mg, about 240-250 mg, about 250-260 mg, about 260-280 mg, about 280-300 mg, about 300-350 mg, about 350-400 mg, about 25 mg, about 50 mg, about 100 mg, about 250 mg, or in an amount within any range defined by any of these values.

In some embodiments, the HIF-PHI or a pharmaceutically acceptable salt thereof is present in each unit pharmaceutical preparation in a percentage relative to the total weight of the pharmaceutical preparation of: about 0.1% (w/w) to about 10% (w/w), about 0.05% (w/w) to about 5% (w/w), about 0.2% (w/w) to about 15% (w/w), about 0.5% (w/w) to about 30% (w/w), about 1% (w/w) to about 25% (w/w), about 2% (w/w), about 3% (w/w), about 4% (w/w), about 5% (w/w), at least about 10% (w/w), at least about 20% (w/w), at least about 50% (w/w), at least about 70% (w/w), at least about 80% (w/w), about 10% (w/w) to about 30% (w/w), about 10% (w/w) to about 20% (w/w), about 20% (w/w) to about 30% (w/w), about 30% (w/w) to about 50% (w/w), about 30% (w/w) to about 40% (w/w), about 40% (w/w) to about 50% (w/w), about 50% (w/w) to about 80% (w/w), about 50% (w/w) to about 60% (w/w), about 70% (w/w) to about 80% (w/w), or about 80% (w/w) to about 90% (w/w), or in a percentage within any range defined by any of these values.

The preparation of the pharmaceutical compositions (or pharmaceutical preparations) can employ methods well-known or commonly used in the art, which generally involve the step of mixing the active ingredient HIF-PHI with pharmaceutically acceptable carriers, adjuvants, or excipients, and optional post-treatment or processing steps (e.g., drying, granulation, encapsulation, etc.).

### 3. Pharmaceutical Kit

A second aspect of the present application provides a pharmaceutical kit, comprising a packaging container, a medication instruction, and a pharmaceutical preparation contained within the packaging container, wherein the pharmaceutical preparation is the pharmaceutical preparation according to the first aspect of the present application, and the medication instruction contains instructions indicating that the pharmaceutical preparation is suitable for oral administration at a dosing interval of once weekly or longer.

The pharmaceutical kit in the present application refers to a finished drug product that meets requirements of relevant regulatory agencies and can be sold in pharmacies for direct selection or purchase by physicians or patients. It typically has an individual package or packaging container, includes the pharmaceutical preparation contained within the packaging container, and is accompanied by a medication instruction. The medication instruction contains specified information required by relevant regulatory agencies including active ingredient, excipients, indications, contraindications, possible side effects, directions for use, precautions, and the like. The medication instruction may be a separate sheet of paper, a label, a booklet contained within the packaging container, or may be instructional text printed directly onto the packaging container. The medication instruction may also be an accompanying electronic medication instruction, such as a separate CD-ROM or a URL or QR code containing address of the medication instruction information online.

Because the pharmaceutical preparation in the present application is a long-acting oral pharmaceutical preparation, its medication instruction shall contain information indicating that the pharmaceutical preparation is permitted or suitable for oral administration at a dosing interval of once weekly or longer.

### 4. Pharmaceutical Use or Treatment Method

A third aspect of the present application provides use of a hypoxia inducible factor-prolyl hydroxylase inhibitor (HIF-PHI) or a pharmaceutically acceptable salt thereof in the manufacture of a long-acting oral medicament for treating chronic anemia, wherein the long-acting oral medicament is suitable for administration at a dosing interval of once weekly or longer.

A fourth aspect of the present application provides a hypoxia inducible factor-prolyl hydroxylase inhibitor (HIF-PHI) or a pharmaceutically acceptable salt thereof for use in orally treating chronic anemia, wherein the oral treatment is administered at a dosing interval of once weekly or longer.

A fifth aspect of the present application provides tuse of a hypoxia inducible factor-prolyl hydroxylase inhibitor (HIF-PHI) or a pharmaceutically acceptable salt thereof for treating chronic anemia, wherein the HIF-PHI or pharmaceutically acceptable salt thereof is orally administered at a dosing interval of once weekly or longer.

A sixth aspect of the present application provides a method for treating chronic anemia, comprising: orally administering to a patient in need thereof a therapeutically effective amount of a hypoxia inducible factor-prolyl hydroxylase inhibitor (HIF-PHI) or a pharmaceutically acceptable salt thereof at a dosing interval of once weekly or longer.

As used herein, the term "patient" refers to all mammals including humans. Examples of patients include humans, cattle, dogs, cats, goats, sheep, mice, pigs, and rabbits.

As used herein, chronic anemia may be selected from the group consisting of idiopathic anemia, chronic renal anemia, anemia induced by cancer chemotherapy, anemia caused by blood loss, anemia caused by menorrhagia in women, iron deficiency anemia, vitamin deficiency anemia, hypoplastic and aplastic anemia, hemolytic anemia, sideroblastic anemia, and anemia caused by hypothyroidism; preferably, it is chronic renal anemia.

The pharmaceutical kit of the present application may also be used to treat complications of anemia or ischemic diseases, such as ischemic cerebrovascular disease, ischemic renal disease (IRD), ischemic cardiomyopathy (ICM), and the like.

When using the pharmaceutical preparation of the present application to treat chronic anemia, a therapeutically effective amount of the active ingredient (i.e., HIF-PHI) is generally administered to the patient. As used herein, the term "therapeutically effective amount" refers to an amount of the active ingredient mentioned in the present application that, when administered to a patient, is effective to delay or eliminate the patient's symptoms or improve the patient's health condition. The specific applied dosage can be determined by a physician based on the patient's specific circumstances. The precise dosage to be employed depends not only on route of administration, condition, severity of the condition to be treated, and various physical factors related to the individual being treated, but may also be determined at the discretion of the healthcare practitioner. In vitro or in vivo assays may optionally be used to help determine optimal dosage ranges.

For example, a compound of general formula I of the present application may be administered to a patient at a dose of about 0.01-4000 mg per week, or 0.05-2000 mg, or 0.1-1000 mg, or 0.1-500 mg, or 9-30 mg, or 9-18 mg, or 18-30 mg. However, the specific dosage used may vary. Those skilled in the art know how to determine the optimal dosage for a particular patient.

In some embodiments, the dose of HIF-PHI per administration may be about 0.01-0.2 mg, about 0.2-0.4 mg, about 0.4-0.6 mg, about 0.6-0.8 mg, about 0.8-1 mg, about 1-1.2 mg, about 1.2-1.4 mg, about 1.4-1.6 mg, about 1.6-1.8 mg, about 1.8-2 mg, about 2-2.2 mg, about 2.2-2.4 mg, about 2.4-2.6 mg, about 2.6-2.8 mg, about 2.8-3 mg, about 3-3.2 mg, about 3.2-3.4 mg, about 3.4-3.6 mg, about 3.6-3.8 mg, about 3.8-4 mg, about 3.9-4.1 mg, about 4-4.2 mg, about 0.2-0.4 mg, about 0.2-0.6 mg, about 0.2-0.8 mg, about 0.2-1 mg, about 0.2-1.2 mg, about 0.2-1.4 mg, about 0.2-1.6 mg, about 0.2-1.8 mg, about 0.2-2.0 mg, 0.2-2.5 mg, about 0.2-3.0 mg, about 0.2-3.5 mg, about 0.2-4.0 mg, about 5-10 mg, about 10-15 mg, about 15-20 mg, about 20-25 mg, about 25-30 mg, about 30-40 mg, about 40-50 mg, about 50-60 mg, about 60-70 mg, about 70-80 mg, about 80-90 mg, about 90-100 mg, about 100-120 mg, about 120-140 mg, about 140-150 mg, about 150-160 mg, about 160-180 mg, about 180-200 mg, about 200-220 mg, about 220-240 mg, about 10-500 mg, about 50-400 mg, about 50-300 mg, about 100-250 mg, about 1-10 mg, about 10-200 mg, about 10-150 mg, about 10-100 mg, about 10-180 mg, about 10-160 mg, about 10-140 mg, about 10-120 mg, about 10-100 mg, about 10-20 mg, about 20-30 mg, about 30-40 mg, about 40-50 mg, about 50-60 mg, about 60-70 mg, about 70-80 mg, about 80-90 mg, about 90-100 mg, about 100-120 mg, about 120-140 mg, about 140-160 mg, about 160-180 mg, about 180-200 mg, about 200-220 mg, about 220-240 mg, about 240-250 mg, about 250-260 mg, about 260-280 mg, about 280-300 mg, about 300-350 mg, about 350-400 mg, about 25 mg, about 50 mg, about 100 mg, about 250 mg, or a dose within any range defined by any value in these doses or dose ranges.

The present application proposes an innovative dosing regimen that breaks the limitations of traditional pharmaceutical theory regarding dosing cycles. According to the dosing regimen described in the present application, patient adherence can be improved when a long-term treatment for patients with chronic renal anemia is performed, especially during the titration period, thereby enhancing therapeutic efficacy and making long-term management of the chronic disease easier.

The following further illustrates the present application in conjunction with examples and drawings; however, these examples do not limit the scope of this application.

### Description of the Drawings

Figure 1 shows a comparison of hemoglobin bar charts for rats in each group on days 0, 7, 14, 21, and 28 from Example 3.
Figure 2 shows a comparison of hemoglobin bar charts for rats in each group on day 28 and the statistical significance between groups from Example 3.

### Examples

The examples described below are part of the embodiments of the present invention, not all of the embodiments. The detailed description of the embodiments of the present invention is not intended to limit the scope of the claimed invention, but merely represents selected embodiments of the present invention. Based on the embodiments of the present invention, all other embodiments obtained by a person of ordinary skill in the art without departing from the principles of the present invention and without creative effort shall fall within the protection scope of the present invention.

For the sake of brevity, some materials, equipment, and method steps conventionally used in the art are not individually specified in the examples. All process methods and analytical testing procedures (and related parameters) not specifically noted in the examples are carried out according to those commonly used by those skilled in the art; and materials, reagents, and equipment not specified with a specific source are conventional laboratory materials and equipment, which can be obtained through commercial channels.

### Example 1

### Synthesis of (6'-hydroxy-8'-oxo-3'-phenyl-8'H-spiro[cyclopentane-1,5'-indolizine]-7'-carbonyl)glycine (1)

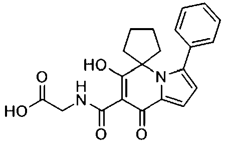

Compound 1 was synthesized according to the method of Example 24 in WO2018205928.

The structure of the compound was confirmed by liquid chromatography-mass spectrometry (LCMS) or nuclear magnetic resonance (NMR). NMR chemical shifts (δ) are expressed in units of parts per million (ppm). NMR was measured using a Bruker-500 type NMR spectrometer, with deuterated dimethyl sulfoxide (dmso-d6), deuterated chloroform (CDCl₃), etc., as a measurement solvent, and tetramethylsilane (TMS) as an internal standard. LCMS was measured using a Shimadzu LCMS-2020 or Thermo UltiMate 3000.

Thin-layer chromatography silica gel plates used were Yantai Huanghai HSGF254 or Yantai Qingdao GF254 silica gel plates from Shandong Province. Column chromatography generally used Yantai Huanghai silica gel (200-300 mesh) from Shandong Province as a carrier.

All starting materials used in this example were purchased from chemical suppliers or could be synthesized by literature methods.

Abbreviations possibly used in this example are as follows:
DMSO-d6: Dimethyl sulfoxide where all six hydrogen atoms are replaced by deuterium
CDCl₃: Deuterated chloroform
CAS: Chemical Abstracts Service registration number
NMR: Nuclear magnetic resonance
LCMS: Liquid chromatography-mass spectrometry
ESI: Electrospray ionization
ppm: Parts per million
δ: NMR chemical shift
TMS: Tetramethylsilane
s: NMR singlet
d: NMR doublet
t: NMR triplet
br: NMR broad peak
CDI: Carbonyldiimidazole
DCC: N,N'-Dicyclohexylcarbodiimide
NBS: N-Bromosuccinimide

The main synthetic steps for Compound 1 were as follows.

### Step 1: 1-(2-Phenyl-1H-pyrrol-1-yl)cyclopentane-1-carboxylic acid (1a)

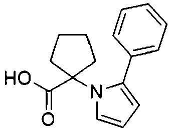

3-(1,3-dioxan-2-yl)-1-phenylpropan-1-one (prepared according to the method described in patent document WO/2011/042477) (500 mg) and 1-amino-cyclopentane-1-carboxylic acid (421 mg) were refluxed in 8 mL of acetic acid for 16 hours. Then, after cooling the reaction mixture, acetic acid was evaporated as much as possible; and then water and ethyl acetate were added for dilution. The resulting organic phase was collected, and the organic phase was washed several times with dilute aqueous sodium chloride solution until the aqueous phase was nearly neutral; then the ethyl acetate layer was dried over anhydrous sodium sulfate, filtered, concentrated, and purified by column chromatography to obtain Compound 1a. LCMS ESI(+): 256 (M+1)⁺. ¹H NMR (500 MHz, DMSO-d6) δ (ppm): 12.95 (br, s, 1H), 7.35-7.33 (m, 3H), 7.25-7.22 (m, 2H), 7.00-6.99 (m, 1H), 6.04 (m, 1H), 5.95 (m, 1H), 2.14-2.11 (m, 2H), 2.01-1.97 (m, 2H), 1.60-1.57 (m, 4H).

### Step 2: Dimethyl 2-(1-(2-phenyl-1H-pyrrol-1-yl)cyclopentane-1-carbonyl)malonate (1b)

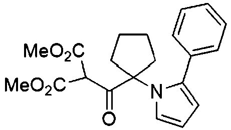

Following the synthetic route for the second step of Example 15 (compound 15b) in WO2018205928, the starting material 15a was replaced with 1a to yield 1b. LCMS ESI(+): 370 (M+1)⁺. ¹H NMR (500 MHz, DMSO-d6) δ 7.28 - 7.22 (m, 3H), 7.20 - 7.15 (m, 2H), 7.11 (dd, J = 3.2, 1.8 Hz, 1H), 6.15 (t, J = 3.3 Hz, 1H), 5.93 (dd, J = 3.5, 1.7 Hz, 1H), 5.13 (s, 1H), 3.56 (s, 6H), 2.06 (d, J = 8.0 Hz, 2H), 1.59 (t, J = 12.2 Hz, 2H), 1.33 (s, 2H), 1.16 (s, 2H).

### Step 3: Methyl 6'-hydroxy-8'-oxo-3'-phenyl-8'H-spiro[cyclopentane-1,5'-indolizine]-7'-carboxylate (1c)

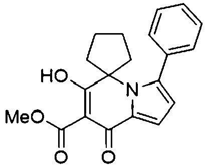

Following the synthetic route for the third step of Example 15 (compound 15c) in WO2018205928, the starting material 15b was replaced with 1b to yield 1c. LCMS ESI(+): 338 (M+1)⁺. ¹H NMR (500 MHz, DMSO-d6) δ 13.84 (s, 1H), 7.54-7.43 (m, 5H), 7.09 (d, J = 4.0 Hz, 1H), 6.35 (d, J = 4.0 Hz, 1H), 3.81 (s, 3H), 2.30-2.21 (m, 2H), 2.10-2.01 (m, 2H), 1.48-1.38 (m, 2H), 1.00-0.89 (m, 2H).

### Step 4: (6'-Hydroxy-8'-oxo-3'-phenyl-8'H-spiro[cyclopentane-1,5'-indolizine]-7'-carbonyl)glycine (1)

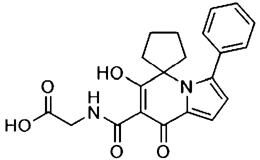

Following the synthetic route for the fourth step of Example 15 (compound 15) in WO2018205928, the starting material 15c was replaced with 1c to yield Compound 1. LCMS ESI(+): 381 (M+1)⁺. ¹H NMR (500 MHz, DMSO-d6) δ (ppm) 17.96 (s, 1H), 12.97 (s, 1H), 9.89-9.87 (d, J = 5.5 Hz, 1H), 7.53-7.46 (m, 5H), 7.08-7.07 (d, 1H), 6.37-6.36 (d, 1H), 4.08-4.07 (d, J = 5.5 Hz, 2H), 2.35-2.20 (m, 2H), 2.18-2.10 (m, 2H), 1.60-1.46 (m, 2H), 0.98-0.85 (m, 2H).

### Example 2: Inhibitory Effect of Comp-1 on HIF PHD-2

In this example and the following examples, Compound 1 prepared in Example 1 was used for testing, and it is referred to as "Comp-1".

Stock solutions of the following reagents were prepared: HEPES buffer (pH 7.4, 50 mM), HIF-1α peptide (sequence: DLDLEMLAPYIPMDDDFQL; Genscript) solution in DMSO/HEPES (0.5 mM; preparation method: first prepare a 20 mM solution of this peptide in DMSO, then dilute it 40-fold with the HEPES buffer), ascorbic acid solution (10 mM), FeSO₄ in hydrochloric acid solution (0.1 mM FeSO₄, 10 µM HCl), 2-oxoglutaric acid solution (2-OG: 1 mM), EDTA solution (pH 8.0; 0.5 M), and eight concentrations of Comp-1/DMSO solutions (concentrations: 4000 µM, 1333 µM, 444.4 µM, 148.1 µM, 49.4 µM, 16.5 µM, 5.5 µM, and 1.82 µM). The following reagents were added to nine Eppendorf tubes: 50 µL HEPES solution, 10 µL ascorbic acid solution, 10 µL FeSO₄ in HCl solution, 10 µL 2-OG solution, 10 µL HIF-1α peptide solution; then 5 µL of each of the eight Comp-1/DMSO solutions and 5 µL of DMSO were added separately; finally, 5 µL of PHD2 enzyme (Active Motif; concentration: 0.2 µg/µL) was added to each tube. Each of the resulting reaction systems was incubated at 30 °C in dark for 2 hours. Finally, 10 µL of EDTA solution was added to each tube to terminate the reaction. The liquid from each reaction system was filtered through a 0.45 µm filter, and then HIF-peptide and hydroxylated HIF-peptide were separated by high-performance liquid chromatography. The concentrations of HIF-peptide and hydroxylated peptide were quantified using a UV absorption detector to calculate their ratio. The IC₅₀ was calculated using GraphPad Prism 6.0 software. IC₅₀ of Comp-1 was 2.36 µM against HIF PHD-2.

### Example 3: Elimination Half-Life of the Compound in Rats

Preparation of Intravenous Injection Formulation: 4.0005 g of HP-β-CD (Shandong Binzhou Zhiyuan Biotechnology Co., Ltd.) was weighed and dissolved in 16.0 mL of water for injection. The resulting mixture was stirred for 20 minutes and the volume was made up to 20 mL to obtain a 20% HP-β-CD solution. 4.93 mg of Comp-1 was weighed, and dissolved in 0.984 mL of ethanol. The resulting mixture was stirred for 13 minutes, and sonicated for 5 minutes. Then, 8.0 + 0.856 mL of 20% HP-β-CD was added, followed by stirring for 4 minutes and sonication for 4 minutes, to obtain 9.84 mL of a clear Comp-1 solution with a final concentration of 0.5 mg/mL.

Preparation of Single-Dose Oral Formulation: 25.3800 g of meglumine (Sigma) was weighed and dissolved in 80 mL of deionized water. The resulting mixture was stirred for 35 minutes and the volume was made up to 130 mL to obtain a 1M meglumine solution. 4.31 mg, 13.54 mg, and 40.59 mg of Comp-1 were weighed separately and then added to 28.676 mL, 27 mL, and 27 mL of the 1M meglumine solution respectively. The resulting mixtures each was stirred for 31 minutes and sonicated for 5 minutes to obtain colorless transparent solutions with Comp-1 concentrations of 0.15 mg/mL, 0.5 mg/mL, and 1.5 mg/mL, respectively.

18 SD rats (half male, half female) were randomly divided into 3 groups (3 males and 3 females per group). Comp-1 was administered by oral gavage at doses of 1.5, 5.0, and 15.0 mg/kg. Blood samples were collected before administration and at 0.25, 0.5, 1, 2, 4, 6, 8, 24, 48, and 72 hours after administration. The oral vehicle was a 1 M meglumine solution in deionized water, and the administration volume was 10 mL/kg for all doses. All collected plasma samples were stored at -75±15°C until analysis. The concentration of Comp-1 in SD rat plasma was determined using liquid chromatography-tandem mass spectrometry with Tolbutamide as an internal standard. The mass spectrometer utilized an electrospray ionization source operating in positive ion mode. The precursor-to-product ion transitions monitored were m/z 381.2→306.3 for Comp-1 and m/z 270.9→155.0 for Tolbutamide. Pharmacokinetic parameters of Comp-1 were calculated by software PhoenixTM WinNonlin version 6.1 using a non-compartmental model. Pharmacokinetic data were calculated using a linear log trapezoidal method with a weighting of 1/Y*Y. Samples with concentrations below the lower limit of quantification were not included in the calculation of pharmacokinetic parameters. Microsoft Excel 2010 was used to calculate the mean values of various parameters, with the mean determined from the pharmacokinetic parameters of each animal. The mean elimination half-lives of Comp-1 in male rats at the doses of 1.5, 5.0, and 15.0 mg/kg were: 6.72 hours, 15.0 hours, and 10.2 hours, respectively. The mean elimination half-lives of Comp-1 in female rats at the doses of 1.5, 5.0, and 15.0 mg/kg were: 12.9 hours, 5.72 hours, and 24.9 hours, respectively. All t1/2 values were significantly less than 33.6 hours.

### Example 4: Effect of Comp-1 Orally Administered Three Times Weekly (tiw) and Once Weekly (qw) on Increasing Hemoglobin in Normal Rats

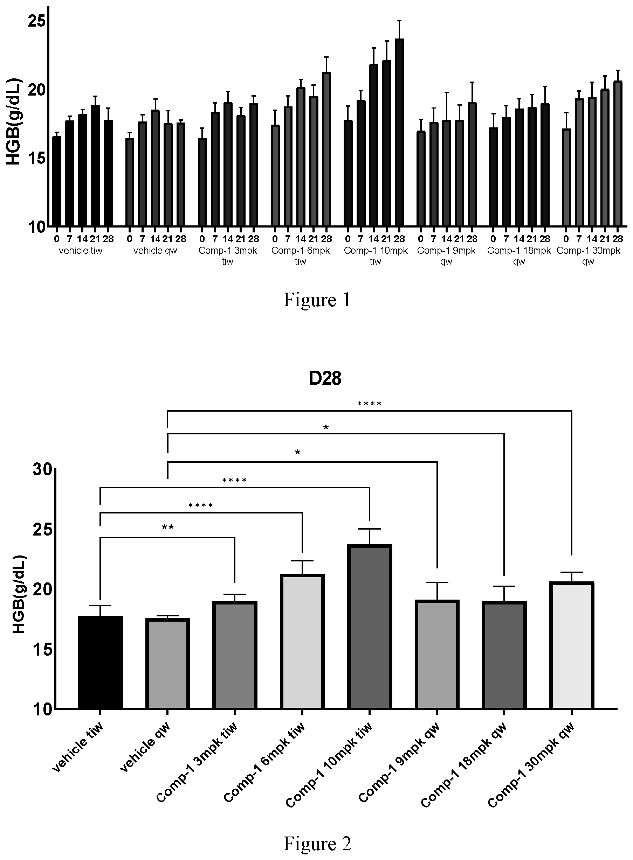

A total of 70 male SD rats, approximately 7 weeks old, were acclimated to the experimental environment for 10 days before the start of the experiment. Throughout the experiment, animals were housed in cages (5 rats per cage) in a conventional animal room with 24-hour continuous central air conditioning, temperature set at 24°C, and a 12-hour light/dark cycle. All rats had free access to food (dry pelleted food) and purified drinking water. The animals were randomly divided into 8 groups: the vehicle tiw group and vehicle qw group each contained 5 rats; Comp-1 3 mpk tiw, 6 mpk tiw, 10 mpk tiw, 9 mpk qw, 18 mpk qw, and 30 mpk qw, each contained 10 rats (mpk = mg/kg). The vehicle was 1 M aqueous meglumine solution. Each rat received oral gavage of the vehicle or the corresponding weight of Comp-1 vehicle solution according to its body weight and group assignment (tiw, qw) for a total of 4 weeks. Blood samples were collected from each rat on days 0, 7, 14, 21, and 28 to measure hemoglobin values (HGB, Hb). After tail snip blood collection, hemoglobin values were measured and read using a hemoglobin detector (HemoCue Hb 201+, produced by Radiometer). Quantitative data results were analyzed statistically using one-way analysis of variance (ANOVA) between groups, followed by Dunnett's multiple comparison method. Two-tailed analysis was used for statistics, and the statistical significance level was set at P < 0.05. The hemoglobin results for rats in each group were shown in Figure 1, Figure 2, and Table 1.

**Table 1: Comparison of Hemoglobin Results in Rats from Each Group on Days 0, 7, 14, 21, and 28**

| **Group** | **N** | | | | | |
|---|---|---|---|---|---|---|
| | | **D0** | **D7** | **D14** | **D21** | **D28** |
| Vehicle tiw | 5 | 16.58±0.28 | 17.7±0.32 | 18.16±0.35 | 18.82±0.67 | 17.74±0.86 |
| Vehicle qw | 5 | 16.44±0.38 | 17.64+0.50 | 18.48±0.81 | 17.52±0.91 | 17.56±0.19 |
| Comp-1 (3 mg/kg, tiw) | 10 | 16.42+0.74 | 18.32±0.70 | 19.06±0.81* | 18.09±0.57* | 18.97±0.56** |
| Comp-1 (6 mg/kg, tiw) | 10 | 17.4±1.07 | 18.76±0.76* | 20.15±0.58**** | 19.49+0.82 | 21.26±1.08**** |
| Comp-1 (10 mg/kg, tiw) | 10 | 17.74±1.06 | 19.21±0.71*** | 21.83±1.18**** | 22.12±1.40*** | 23.69±1.31**** |
| Comp-1 (9 mg/kg, qw) | 10 | 16.98±0.82 | 17.57±1.07 | 17.77±2.01 | 17.73±1.15 | 19.09±1.44# |
| Comp-1 (18 mg/kg, qw) | 10 | 17.2±1.00 | 17.95+0.88 | 18.57+0.76 | 18.72±0.92# | 18.99±1.23# |
| Comp-1 (30 mg/kg, qw) | 10 | 17.13±1.17 | 19.33±0.56#### | 19.44±1.07 | 20.04±0.93### | 20.62±0.76#### |

| | | | | | | |
|---|---|---|---|---|---|---|
| ****P < 0.0001 vs vehicle tiw. ***P < 0.001 vs vehicle tiw. **P < 0.01 vs vehicle tiw. *P < 0.05 vs vehicle tiw. ####P < 0.0001 vs vehicle qw. ###P < 0.001 vs vehicle qw. ##P < 0.01 vs vehicle qw. #P < 0.05 vs vehicle qw. | | | | | | |

According to the results shown in Figure 1, Figure 2, and Table 1, on day 28, compared with the rats orally administered the vehicle once weekly (qw), the hemoglobin levels in the three groups of rats administered the drug once weekly (qw) were all statistically significantly increased, and the increase in hemoglobin could be maintained. Furthermore, in the treatment of non-dialysis-dependent chronic kidney disease (NDD CKD) anemia, the most critical factor is not efficacy but cardiovascular safety. That is, it is not that the higher and faster the hemoglobin increase. Instead, during the initial dose-fixing period of treatment, a certain increase is acceptable, and a slower rate of hemoglobin increase is safer than a faster one. Comparing groups with the same total weekly dose on day 28 (Comp-1 6 mg/kg, tiw vs. Comp-1, 18 mg/kg, qw; Comp-1 10 mg/kg, tiw vs. Comp-1, 30 mg/kg, qw) as shown in Table 1, it can be found that the absolute increase in hemoglobin was lower and the rate of increase was slower in the qw groups compared to the tiw groups.

### Example 5: Effect of Comp-1 Orally Administered Once Daily (QD), Three Times Weekly (tiw), and Once Weekly (qw) on Increasing Hemoglobin in Normal Mice

This experiment evaluated the effects of different dosing frequencies of the test compound Comp-1 at the same weekly dose on hemoglobin and various routine hematological parameters in male C57BL/6J mice.

After acclimatization feeding, on Day 0, submandibular vein blood was collected from 92 male mice aged 6-8 weeks for routine hematological analysis. Based on the Day 0 Hb (hemoglobin) results, 80 mice were selected for use in this experiment and divided into groups as follows: Vehicle (QD, 5 mice), Comp-1 (4.5mg/kg, QD, 5 mice), Comp-1 (10.5mg/kg, TIW, 5 mice), Comp-1 (31.5mg/kg, QW, 5 mice), Comp-1 (9mg/kg, QD, 15 mice), Comp-1 (21mg/kg, TIW, 15 mice), Comp-1 (63mg/kg, QW, 15 mice), Comp-1 (15mg/kg, QD, 5 mice), Comp-1 (35mg/kg, TIW, 5 mice), and Comp-1 (105mg/kg, QW, 5 mice). On Day 14 and Day 28, submandibular vein blood was collected for routine hematological analysis. Quantitative data results were analyzed statistically using one-way analysis of variance (ANOVA) between groups, followed by Dunnett's multiple comparison method. Two-tailed analysis was used for statistics, and the statistical significance level was set at P < 0.05. The hemoglobin results for mice in each group were shown in Table 2. On Day 14 and Day 28, the mean hemoglobin values of all groups receiving Comp-1 were significantly different from the Vehicle control group, P < 0.001.

**Table 2: Comparison of Hemoglobin Levels in Mice from Each Group on Days 0, 14, and 28**

| Groups | Total weekly doses | N | Hb (g/dL) Day 0 | Hb (g/dL) Day 14 | Hb (g/dL) Day 28 |
|---|---|---|---|---|---|
| Vehicle (QD) | 0 mg/kg | 5 | 15.3±0.51 | 15.6±0.23 | 16.0±0.51 |
| Comp-1 (4.5 mg/kg, QD) | 31.5 mg/kg | 5 | 15.2±0.61 | 18.2±0.52 | 19.7±0.53 |
| Comp-1 (10.5 mg/kg, TIW) | 31.5 mg/kg | 5 | 15.3±0.66 | 17.9±0.54 | 19.2±0.22 |
| Comp-1 (31.5 mg/kg, QW) | 31.5 mg/kg | 5 | 15.3±0.48 | 16.7±0.40 | 17.2±0.52 |
| Comp-1 (9.0 mg/kg, QD) | 63.0 mg/kg | 15 | 15.3±0.51 | 20.6±0.44 | 23.1±0.80 |
| Comp-1 (21.0 mg/kg, TIW) | 63.0 mg/kg | 15 | 15.3±0.49 | 19.7±0.77 | 22.0±0.11 |
| Comp-1 (63.0 mg/kg, QW) | 63.0 mg/kg | 15 | 15.3±0.51 | 17.4±0.62 | 18.5±0.74 |
| Comp-1 (15.0 mg/kg, QD) | 105.0 mg/kg | 5 | 15.3±0.48 | 20.1±0.54 | 22.8±0.29 |
| Comp-1 (35.0 mg/kg, TIW) | 105.0 mg/kg | 5 | 15.3±0.44 | 18.9±0.11 | 22.4±0.57 |
| Comp-1 (105.0 mg/kg, QW) | 105.0 mg/kg | 5 | 15.3±0.50 | 19.0±0.40 | 20.0±0.48 |

According to the results in Table 2 above, at the same total weekly dose, mice dosed once weekly (qw) were similar to those dosed once daily or three times weekly, and likewise showed a significant increase in hemoglobin that could be maintained long-term, with statistical significance compared to the control group. Moreover, compared to mice dosed once daily or three times weekly, the absolute magnitude of hemoglobin increase and the rate of increase were smaller with once-weekly dosing, which is more conducive to improving safety of chronic kidney disease anemia treatment.

### Example 6: Elimination Half-Life of Comp-1 in Humans After Single and Multiple Oral Doses

In a Phase 1, randomized, double-blind, placebo-controlled, single-dose and multiple-dose clinical study, a total of 46 healthy volunteers were assigned to one of six dose groups (1 mg, 4 mg, 10 mg, 20 mg, 30 mg, 50 mg) in a single-dose phase. In each dose group, 2 volunteers received a matching placebo. In the 1 mg dose group, 4 volunteers received a single dose of Comp-1 and in the remaining dose groups, 6 volunteers each received a single dose of Comp-1. Blood samples were collected from volunteers within 1 hour before dosing and at 0.5, 1, 1.5, 2, 3, 4, 5, 6, 7, 8, 10, 12, 24, 48, and 72 hours after dosing for pharmacokinetic (PK) analysis.

A total of 32 healthy volunteers were assigned to one of four dose groups (4 mg, 10 mg, 20 mg, 30 mg) in a multiple-dose phase. In each dose group, 2 volunteers received a matching placebo and 6 volunteers received Comp-1 once daily for 10 consecutive days. Blood samples for PK analysis were collected from volunteers within 1 hour before dosing and at 0.5, 1, 1.5, 2, 3, 4, 5, 6, 7, 8, 10, 12, and 24 hours after dosing on the first dosing day (i.e., Day 1), as well as within 1 hour before dosing and at 0.5, 1, 2, 3, 4, 5, 6, 7, 8, 10, 12, 24, 48, and 72 hours after dosing on Days 6, 7, 8, and 10.

In the single-dose phase, among volunteers receiving a single dose of Comp-1, peak drug concentration (Cₘₐₓ) was reached in each dose group at 3-4.5 hours (median Tₘₐₓ); exposure (Cₘₐₓ, AUC₀₋ₜ, and AUC_{0-inf}) increased in a dose-proportional manner; the mean elimination half-life (t_{1/2}) was 13.06-19.70 hours, with no clear dose dependence; and mean clearance (C_{L/F}) showed no dose-related trend.

In the multiple-dose phase, after volunteers received the first dose of Comp-1 on Day 1, peak drug concentration (Cₘₐₓ) was reached in each dose group at 2-3 hours (median Tₘₐₓ), with no significant differences between dose levels. Comp-1 exposure (Cₘₐₓ, AUC₀₋ₜ, and AUC_{0-inf}) after the first dose increased with dose; and the mean half-life was 10.14-19.43 hours, showing no trend with dose. C_{L/F} also showed no apparent trend with dose. After 10 consecutive days of dosing, the median Tₘₐₓ for each dose group was 3-4 hours; Comp-1 exposure (C_{ss,max}, AUC_{0-τ}, AUC₀₋ₜ, and AUC_{0-inf}) increased in a dose-proportional manner; the mean elimination half-life was 12.41-15.75 hours, showing no dose-related trend; and mean clearance (C_{L/F}) showed no dose-related trend. These elimination half-lives were all significantly shorter than 33.6 hours.

### Example 7: Results of a Phase 2 Clinical Trial of Comp-1 in Patients with Chronic kidney disease Anemia

A Phase 2, randomized, double-blind, placebo-controlled clinical study of Comp-1 in patients with chronic kidney disease anemia included a total treatment period of 13 weeks, divided into an initial 5-week dose-fixing period and a subsequent 8-week dose adjustment period (also referred to as the titration period). A total of 113 patients with renal anemia were initially randomized into the dose-fixing period. During the dose-fixing period, patients with renal anemia received Comp-1 8 mg, 12 mg, or 16 mg three times weekly (TIW), or a matching placebo. At the Week 6 study visit, a total of 44 eligible patients underwent a second randomization into the dose adjustment period (titration period). Patients in the Comp-1 groups had a 50% chance of having their dosing frequency switched from the original TIW to once weekly (QW). The starting dose in this phase was the same as the dose during the dose-fixing period and could be adjusted based on weekly hemoglobin values to maintain hemoglobin within the target range of 10.0-11.0 g/dL, inclusive. To maintain blinding, patients in the placebo group underwent pseudo-randomization and continued to receive matching placebo. During the study, patients were visited weekly to measure the primary efficacy indicator, i.e. hemoglobin level, and safety comparisons were made for patients entering the titration period according to dosing frequency (three times weekly (TIW) dosing vs. once weekly (QW) dosing).

The primary efficacy objective of the study was to compare the mean rate of hemoglobin increase (g/dL/week) at the end of the dose-fixing period (i.e., after 5 weeks of dosing) between the three Comp-1 dose groups and the placebo group. The study results showed that the mean rate of hemoglobin increase in each Comp-1 dose group showed a dose-related increase: 0.2839 g/dL/week in the 8 mg group, 0.3893 g/dL/week in the 12 mg group, and 0.4817 g/dL/week in the 16 mg main group, all higher than -0.0382 g/dL/week in the placebo group, with statistical significance (all p-values < 0.0001). In the analysis of hemoglobin during the adjustment period (titration period), at Week 14 (i.e., at the end of the titration period or after 13 weeks of dosing), the mean levels of hemoglobin in the pooled Comp-1 TIW group and QW group were 10.74 g/dL and 10.09 g/dL, respectively, both maintained within the study target range of 10.0-11.0 g/dL inclusive, while the mean level of hemoglobin in the placebo group was 9.57 g/dL. During the later part of the adjustment period (titration period), i.e., Weeks 10-14, the mean levels of hemoglobin were >10.0 g/dL in all dose frequency groups including the placebo group: 10.73 g/dL and 10.77 g/dL in the pooled Comp-1 TIW and QW groups, respectively, and 10.26 g/dL in the placebo group. The change in hemoglobin level from baseline was 1.60 g/dL and 1.63 g/dL in the pooled Comp-1 TIW and QW groups, respectively, and 0.48 g/dL in the placebo group. This indicated that patients in the placebo group, who underwent second randomization screening, could maintain hemoglobin within the study target range during the adjustment period even without relevant treatment and with only a small increase (or fluctuation) in hemoglobin levels, whereas the performance in increasing and maintaining hemoglobin levels was comparable between the pooled Comp-1 TIW and QW groups. In the analysis of hemoglobin throughout the entire adjustment period (titration period), i.e., Weeks 6-14, the mean hemoglobin levels in the pooled Comp-1 TIW and QW groups were 10.84 g/dL and 10.91 g/dL, respectively, compared to 10.23 g/dL in the placebo group. The change in hemoglobin from baseline was 1.71 g/dL and 1.78 g/dL in the pooled Comp-1 TIW and QW groups, respectively, and 0.45 g/dL in the placebo group, again demonstrating comparable efficacy in increasing and maintaining hemoglobin levels between the TIW and QW groups. Throughout the entire 13-week treatment period, the cumulative percentage of patients achieving the target hemoglobin range of 10.0-11.0 g/dL inclusive and an increase of ≥1.0 g/dL from baseline was similar between the pooled Comp-1 TIW and QW groups: 100% and 90%, respectively, compared to 40% in the placebo group. Table 3 below provides a safety comparison for patients entering the titration period according to dosing frequency.

**Table 3: Safety Comparison According to Dosing Frequency During the Titration Period**

| **TEAE for Comp 1** | | **Treatment-related TEAE for Comp-1** | |
|---|---|---|---|
| **Pooled TIW** | **Pooled QW** | **Pooled TIW** | **Pooled QW** |
| **N=19** | **N=20** | **N=19** | **N=20** |
| **n (%), NE** | **n (%), NE** | **n (%), NE** | **n (%), NE** |
| 14 (73.7%), **39** | 14 (70.0%), **18** | 2 (10.5%), **2** | 1 (5.0%), **1** |

TEAE: treatment emergent adverse event; Treatment-related TEAE: treatment emergent adverse event considered related to treatment; N: number of patients; NE: total number of adverse events occurring; n: number of patients experiencing adverse events.

From Table 3, it can be seen that after entering the titration period, the QW (once weekly) dosing frequency resulted in lower percentages of patients experiencing both TEAEs and treatment-related TEAEs compared to the TIW (three times weekly) dosing frequency. This was particularly evident in the total number of adverse events (NEs), where the QW (once-weekly) dosing frequency showed fewer events. Considering that currently marketed HIF-PHI inhibitors for treating CKD anemia are dosed either once daily or three times weekly, what is surprising from the results in Table 3 is that patients with the less dosing frequency experienced fewer adverse events.

According to Example 6, the elimination half-life of Comp-1 is much shorter than one-fifth of a week, i.e., 33.6 hours. However, during the dose adjustment period (titration period) in the treatment of patients with chronic kidney disease anemia, therapeutic effects can still be achieved even with once-weekly oral administration. Specifically, it maintains patients' hemoglobin within the expected target range, and the once-weekly dosing frequency results in fewer adverse events compared to a three-times-weekly dosing regimen. Therefore, this represents an innovative dosing regimen and frequency that produces unexpectedly favorable therapeutic outcomes.

Although embodiments of the present application have been illustrated and described, it is not intended that these examples illustrate and describe all possible forms of the present application. Rather, the words used in the specification are words of description rather than limitation, and it is understood that various changes may be made without departing from the spirit and scope of the present application.

## Claims

1. A long-acting oral pharmaceutical preparation, comprising a hypoxia inducible factor-prolyl hydroxylase inhibitor (HIF-PHI) or a pharmaceutically acceptable salt thereof, and one or more pharmaceutically acceptable carriers, adjuvants, or excipients, wherein the long-acting oral pharmaceutical preparation is suitable for administration at a dosing interval of once weekly or longer.

2. A pharmaceutical kit, comprising a packaging container, a medication instruction, and a pharmaceutical preparation contained within the packaging container, wherein the pharmaceutical preparation comprises a hypoxia inducible factor-prolyl hydroxylase inhibitor (HIF-PHI) or a pharmaceutically acceptable salt thereof, and one or more pharmaceutically acceptable carriers, adjuvants, or excipients, and the medication instruction contains information indicating that the pharmaceutical preparation is suitable for oral administration at a dosing interval of once weekly or longer.

3. Use of a hypoxia inducible factor-prolyl hydroxylase inhibitor (HIF-PHI) or a pharmaceutically acceptable salt thereof in the manufacture of a long-acting oral medicament for treating chronic anemia, wherein the long-acting oral medicament is suitable for administration at a dosing interval of once weekly or longer.

4. A method for treating chronic anemia, comprising: orally administering to a patient in need thereof a therapeutically effective amount of a hypoxia inducible factor-prolyl hydroxylase inhibitor (HIF-PHI) or a pharmaceutically acceptable salt thereof at a dosing interval of once weekly or longer.

5. The pharmaceutical preparation according to claim 1, the pharmaceutical kit according to claim 2, the use according to claim 3, or the method according to claim 4, wherein the HIF-PHI or a pharmaceutically acceptable salt thereof has a t1/2 of less than 33.6 hours, less than 24 hours, less than 16 hours, or less than 10 hours.

6. The pharmaceutical preparation according to claim 1, the pharmaceutical kit according to claim 2, the use according to claim 3, or the method according to claim 4, wherein the chronic anemia is selected from the group consisting of idiopathic anemia, chronic kidney disease anemia, anemia induced by cancer chemotherapy, anemia caused by blood loss, anemia caused by menorrhagia in women, iron deficiency anemia, vitamin deficiency anemia, hypoplastic and aplastic anemia, hemolytic anemia, sideroblastic anemia, and anemia caused by hypothyroidism; preferably, chronic kidney disease anemia.

7. The pharmaceutical preparation according to claim 1, the pharmaceutical kit according to claim 2, the use according to claim 3, or the method according to claim 4, wherein the HIF-PHI is selected from the group consisting of Roxadustat, Daprodustat, Vadadustat, Molidustat, Enardustat, Desidustat, HIF117 (SSS17), HEC53856, and DDO-3055.

8. The pharmaceutical preparation according to claim 1, the pharmaceutical kit according to claim 2, the use according to claim 3, or the method according to claim 4, wherein the HIF-PHI is Comp-1.

9. The pharmaceutical preparation according to claim 1, the pharmaceutical kit according to claim 2, the use according to claim 3, or the method according to claim 4, wherein the HIF-PHI is a compound of formula I: 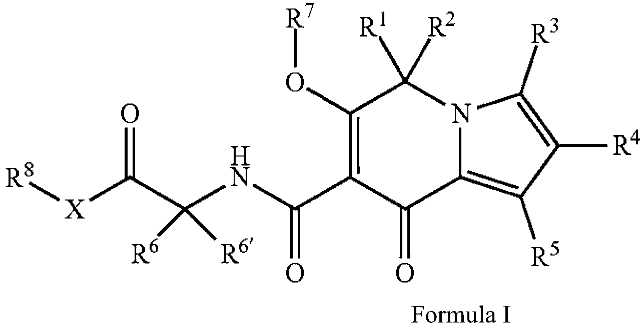 wherein:
R¹ and R² are independently selected from the group consisting of cyano, alkyl, heterocyclyl, alkenyl, alkynyl, aryl, heteroaryl, and acyl; wherein the alkyl, heterocyclyl, alkenyl, alkynyl, aryl, heteroaryl, and acyl are optionally substituted by one or more substituents independently selected from the group consisting of halogen, cyano, hydroxyl, amino, carboxyl, acyl, alkyl, heterocyclyl, alkenyl, alkynyl, aryl, heteroaryl, =O, =S, SH, R¹⁰O-, R¹⁰S-, R¹⁰(O=)S-, and R¹⁰(O=)₂S-, in which R₁₀ is alkyl, heterocyclyl, alkenyl, alkynyl, aryl, or heteroaryl; or R₁ and R₂ are taken together to form a ring;
R³ is selected from the group consisting of alkyl, heterocyclyl, alkenyl, alkynyl, aryl, and heteroaryl;
R⁴ and R⁵ are hydrogen;
R⁶ and R⁶' are hydrogen;
R⁷ is hydrogen;
R⁸ is hydrogen or alkyl; and
X is an oxygen atom.

10. The pharmaceutical preparation, pharmaceutical kit, use, or method according to claim 9, wherein in the compound of formula I, R¹ and R² together with the C atom to which they are attached form a cyclopentane ring (i.e., R¹ and R² are -CH₂CH₂CH₂CH₂-), R³ is phenyl, R⁴ and R⁵ are hydrogen, R⁶ and R⁶' are hydrogen, R⁷ is hydrogen, R⁸ is hydrogen, and X is an oxygen atom, that is, the compound of formula I is (6'-hydroxy-8'-oxo-3'-phenyl-8'H-spiro[cyclopentane-1,5'-indolizine]-7'-carbonyl)glycine.
